(19) Europäisches Patentamt / European Patent Office / Office européen des brevets

(11) **EP 2 363 203 B1**

(12) **EUROPEAN PATENT SPECIFICATION**

(45) Date of publication and mention of the grant of the patent:
**25.03.2020 Bulletin 2020/13**

(21) Application number: **09828939.0**

(22) Date of filing: **09.10.2009**

(51) Int Cl.:
**B01J 8/02** (2006.01)      **C07C 27/14** (2006.01)
**C07C 45/35** (2006.01)      **C07C 47/22** (2006.01)
**C07C 51/25** (2006.01)      **C07C 57/05** (2006.01)
**C07D 301/08** (2006.01)      **C07D 303/04** (2006.01)
**C07B 61/00** (2006.01)

(86) International application number:
**PCT/JP2009/067662**

(87) International publication number:
**WO 2010/061690 (03.06.2010 Gazette 2010/22)**

(54) **METHOD OF REACTION USING HEAT-EXCHANGE TYPE REACTOR**

REAKTIONSVERFAHREN MIT EINEM REAKTOR VOM WÄRMETAUSCHERTYP

PROCÉDÉ DE RÉACTION UTILISANT UN RÉACTEUR DE TYPE ÉCHANGEUR DE CHALEUR

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.11.2008   JP 2008299895**
              **26.12.2008   JP 2008334079**

(43) Date of publication of application:
**07.09.2011   Bulletin 2011/36**

(60) Divisional application:
**18188789.4 / 3 431 175**

(73) Proprietor: **Mitsubishi Chemical Corporation**
**Tokyo 100-8251 (JP)**

(72) Inventors:
• **ISOGAI Shinji**
  **Yokkaichi-shi**
  **Mie 510-8530 (JP)**
• **JINNO Kimikatsu**
  **Yokkaichi-shi**
  **Mie 510-8530 (JP)**
• **SAKAKURA Yasuyuki**
  **Yokkaichi-shi**
  **Mie 510-8530 (JP)**
• **ABE Yoshimune**
  **Yokkaichi-shi**
  **Mie 510-8530 (JP)**
• **YADA Shuhei**
  **Tokyo 108-0014 (JP)**

(74) Representative: **Vossius & Partner**
**Patentanwälte Rechtsanwälte mbB**
**Siebertstrasse 3**
**81675 München (DE)**

(56) References cited:
EP-A1- 1 063 222      EP-A1- 1 526 123
EP-A1- 1 577 001      JP-A- 61 054 229
JP-A- 2003 001 094    JP-A- 2004 167 448
JP-A- 2004 202 430    JP-A- 2004 202 430
JP-A- 2005 320 315    JP-A- 2006 272 192
JP-A- 2008 246 284

Remarks:
The file contains technical information submitted after the application was filed and not included in this specification

EP 2 363 203 B1

**Description**

TECHNICAL FIELD

The present invention relates to a method of reaction using a heat-exchange -type reactor.

Further disclosed is a method of packing a packing material, e.g., a particulate catalyst or inert particles, into the space between heat-transfer plates in a plate-type reactor.

BACKGROUND ART

Shell-and-tube reactors, for example, are known as reactors for use in gas-phase reactions which are exothermic or endothermic and for which particulate solid catalysts are used, such as the gas-phase catalytic oxidation reaction of propane, propylene, or acrolein (see, for example, patent document 1). Also known are plate-type reactors including a reaction vessel for reacting a gaseous raw material therein and heat-transfer tubes and further including a plurality of heat-transfer plates disposed side-by-side within the reaction vessel and a device for supplying a heat medium to the heat-transfer tubes. The reaction vessel is a vessel in which the gas fed passes through the space between adjacent heat-transfer plates and is then discharged. The heat-transfer plates include the multiple heat-transfer tubes, which have been connected to each other at the periphery or edges of the sectional shape. The spaces between the adjacent heat-transfer plates are packed with a catalyst (see, for example, patent document 2).

Shell-and-tube reactors are generally used frequently. However, plate-type reactors, in particular, are superior in the ability to efficiently produce a large quantity of a product through the gas-phase reaction because this type of reactor has a plurality of catalyst layers each formed in the space between adjacent heat-transfer plates and is excellent in contact between each heat-transfer plate and the catalyst.

In shell-and-tube reactors, the temperatures of the reaction layers can be regulated, for example, by packing catalysts differing in particle diameter, diluting a catalyst with an inert substance, or separately controlling the temperatures of the respective catalyst layers. In plate-type reactors, the thickness of each catalyst layer can be regulated, for example, by regulating the distance between the plates and the size of the heat-transfer tubes serving as heat-medium passages, and the temperature of each catalyst layer can be regulated by regulating the flow rate of the heat medium.

For efficiently yielding a product, i.e., for obtaining a larger amount of a product per unit catalyst volume amount (volume of the catalyst), in such a reactor, it is necessary to introduce a raw-material gas in a large amount. However, there has been a problem that when a large amount of a raw-material gas is introduced into the reactor, the reactor comes to have an increased differential pressure and this increased differential pressure in the reactor accelerates a combustion reaction in the oxidation reaction to reduce product yield. In addition, the introduction of a large amount of a raw-material gas into the reactor necessitates a larger amount of energy for compressing the raw-material gas and, hence, has been undesirable also from the standpoint of profitability.

Multitubular reactors and plate-type reactors are known as reactors for obtaining a reaction product from a gaseous raw material through a gas-phase catalytic reaction in the presence of a particulate catalyst. Known examples of the plate-type reactors, of these two types of reactors, include a plate-type reactor which has a reaction vessel for reacting a gaseous raw material therein and a plurality of heat-transfer plates disposed side-by-side within the reaction vessel and in which the heat-transfer plates each include a plurality of heat-transfer tubes connected to each other at the periphery or edges of the sectional shape, the heat-transfer plates having been arranged in the reaction vessel so that the heat-transfer tubes are connected in vertical directions (see, for example, patent document 2).

In the multitubular reactors, a particulate catalyst is packed into the reaction tubes. In the multitubular reactors, the catalyst is packed in relatively thin tubes and, hence, is apt to be sufficiently evenly packed in the reaction tubes regardless of the flowability thereof.

In the plate-type reactors, on the other hand, a particulate catalyst is packed into the spaces between adjacent heat-transfer plates. In such plate-type reactor, since a catalyst is packed into the spaces between the heat-transfer plates, the catalyst being packed flows in the axial direction for the heat-transfer tubes. Because of this, when a catalyst is charged into each space between heat-transfer plates from a plurality of sites arranged in the axial direction for the heat-transfer tubes, the catalyst is not evenly packed in the space between heat-transfer plates, depending on the intervals between the charging sites. There are hence cases where the position of the top of the packing layer in one space fluctuates considerably. There is a fear that such fluctuations may influence reaction control when the plate-type reactor is used in gas-phase catalytic reactions.

Patent document 3 relates to a process for producing phthalic anhydride through gas-phase catalytic oxidation of ortho-xylene, or naphthalene, or both with an oxygen-containing gas using one or more fixed bed reactors, wherein said gas-phase catalytic reaction is performed in three or more individual catalytic layers; the conversion rate from ortho-xylene, or naphthalene, or both at an outlet of a first catalytic layer is 30% to 70%; and the conversion rate from ortho-xylene, or naphthalene, or both at an outlet of a second catalytic layer is 70% or more.

Patent document 4 relates to a catalytic gas phase oxidation reaction, which is a catalytic gas phase oxidation reaction with molecular oxygen or a molecular-oxygen-containing gas by using a fixed-bed multitubular reactor packed with catalysts; with the catalytic gas phase oxidation reaction being characterized in that: a catalyst-packed layer of each reaction tube of the reactor is divided into at least two reaction zones in a tubular axial direction; and the packing of the catalysts is such that the occupation volumes of the catalysts differ between at least two of the reaction zones and that an inert substance molding is mixed in at least one of the reaction zones.

Patent document 5 relates to a plate type catalytic reactor characterized in that a plurality of pairs of heat transfer plates, each formed of two sheets of corrugated plates joined to each other and each having a plurality of heat transfer medium flow passages, are arranged so that projected surface parts and recessed surface parts of the corrugated plates of adjacent heat transfer plates are opposed to each other to form a catalyst layer.

PRIOR-ART DOCUMENTS

PATENT DOCUMENTS

Patent Document 1: JP-A-2003-267912
Patent Document 2: JP-A-2004-202430
Patent Document 3: EP 1 063 222 A1
Patent Document 4: EP 1 526 123 A1
"Patent Document 5: EP 1 577 001 A1

SUMMARY OF THE INVENTION

PROBLEMS THAT THE INVENTION IS TO SOLVE

A first object of the invention, which eliminates the problems described above, is to provide a method of reaction in which even when a large amount of a raw-material gas is introduced into a reactor in order to efficiently yield a product, the reactor can be inhibited from increasing in differential pressure and such raw-material introduction does not result in a decrease in product yield.

A second object is to provide a method in which when a particulate packing material is packed into a plate-type reactor, the packing material can be evenly packed into the spaces between the heat-transfer plates.

MEANS FOR SOLVING THE PROBLEMS

The present inventors diligently made investigations in order to accomplish the first object. As a result, they have found that the yield of a product does not decrease when use is made of a reactor in which the difference in pressure between the raw-material inlet and outlet of the reactor packed with a catalyst is not larger than a given value when air is passed therethrough at a normal-state space velocity per unit catalyst layer volume regulated to or below a specific value. A first aspect of the invention has been thus completed.

Specifically, the factors which affect pressure loss in a reactor equipped with a catalyst layer and operated in a gas passing state include the following conditions relating to the catalyst: the area of contact between the catalyst and the passing gas, which is governed by the surface shape of the catalyst and the particle diameter of the catalyst; porosity in the reactor packed with the catalyst, the porosity governing the liner velocity of the passing gas; and the degree of the clogging of passing-gas passages attributable to the breakage or powdering of the catalyst which occur when the catalyst, during packing, collides against the surfaces of the heat-transfer tube walls and heat-transfer plates which form the catalyst layer and against other substances. Other factors affecting pressure loss, in the case of plate-type reactors, are: the recesses and protrusions of the catalyst-layer-forming heat-transfer plates, which are due to the variety of the shape of the heat-transfer plates characteristics of the reactors; and the length of the gas passages which is attributable to the curvature of the catalyst layers. Still other factors, in the case of both shell-and-tube reactors and plate-type reactors, include: the superficial velocity of the passing gas, which is influenced by the average thickness of the catalyst layers (shell-and-tube reactors) and the thickness of the catalyst layers (plate-type reactors); and the time period of contact with the passing gas, which is influenced by the height of the catalyst layers.

The present inventors have found that when reference conditions are fixed in order to collectively evaluate those catalytic factors in pressure loss and those reactor-shape factors in pressure loss, which hold regardless of the type of reactors, i.e., shell-and-tube reactors or plate-type reactors, then a reactor can be evaluated for pressure loss in a reaction state and for reaction results. With respect to each of a vacant state including no catalyst layer and a packed state including catalyst layers, different space velocities respectively for the two states were fixed as reference conditions involving air. Conditions for evaluating the performance of a reactor were thus determined, and the first aspect of the invention has been completed.

Namely, the first aspect of the invention is defined in the claims. Further disclosed is

(1) a method of reaction which comprises using a reactor which is equipped with a catalyst layer for reacting a raw-material gas fed and is capable of cooling and heating through heat exchange with the catalyst layer, wherein the reactor, in the state of including no catalyst layer, has a differential pressure of 50 Pa or lower when air having ordinary temperature is introduced into the reactor at a space velocity, in terms of normal-state velocity per unit catalyst layer volume, of 7,200 (l/hr) while regulating the reactor outlet pressure to ordinary pressure and without conducting heating with a heat medium,
the catalyst layer having been divided into a plurality of reaction zones arranged from a raw-material gas inlet to a raw-material gas outlet, the multiple reaction zones having been disposed so that the porosity thereof, as measured at the time of catalyst packing, decreases from the raw-material gas inlet toward the raw-material gas outlet.
(2) The method of reaction preferably is a method wherein the reactor to be used is a plate-type reactor which is equipped with a catalyst layer formed between adjacent heat-transfer plates and in which a raw-material gas fed passes through the space between the adjacent heat-transfer plates and is then discharged, a catalyst layer being formed between the adjacent heat-transfer plates.
(3) The method of reaction preferably is a method wherein the reactor to be used is a plate-type reactor in which the catalyst layer formed between the adjacent heat-transfer plates has been divided into a plurality of reaction zones arranged from the raw-material gas inlet toward the raw-material gas outlet, and the multiple reaction zones increase in catalyst layer thickness, in terms of thickness in the direction of the length of the space between the adjacent heat-transfer plates, from the raw-material gas inlet toward the raw-material gas outlet,
the ratio of the particle diameter (D) of the catalyst to be packed into the space between the adjacent heat-transfer plates to the minimum spacing (d) between the adjacent heat-transfer plates (D/d) satisfying 0.9>D/d>0.1.
(4) The method of reaction preferably is a method wherein the catalyst to be packed is a catalyst which, in a bulk state, has a porosity of 60% or lower.
(5) The method of reaction preferably is a method wherein a raw-material gas is introduced into the reactor having a catalyst layer at a space velocity of from 1,100 (1/hr) to 4,200 (l/hr), in terms of normal-state velocity per unit volume of the catalyst layer, to react the gas.
(6) The method of reaction preferably is a method wherein a raw-material gas is introduced into the reactor having a catalyst layer at a space velocity of from 1,800 (l/hr) to 4,200 (l/hr), in terms of normal-state velocity per unit volume of the catalyst layer, to react the gas.
(7) The method of reaction preferably is a method wherein the reactor to be used is a reactor which, in the state of being equipped with a catalyst layer, has a differential pressure of 15 kPa or lower when air having ordinary temperature is introduced into the reactor at a space velocity, in terms of normal-state velocity per unit volume of the catalyst layer, of 1,800 (l/hr) while regulating the reactor outlet pressure to ordinary pressure and without conducting heating with a heat medium.
(8) The method of reaction preferably is a method for use in: producing (meth)acrolein and (meth)acrylic acid from propylene or isobutylene as a raw material; producing (meth)acrylic acid from (meth)acrolein as a raw material; producing ethylene oxide by oxidizing ethylene; producing either at least one unsaturated aliphatic aldehyde having 3 to 4 carbon atoms or at least one unsaturated fatty acid having 3 to 4 carbon atoms or both of these two kinds of compounds by oxidizing at least one member selected from the group consisting of hydrocarbons having 3 to 4 carbon atoms, tertiary butanol, and unsaturated aliphatic aldehydes having 3 to 4 carbon atoms; producing maleic acid from an aliphatic hydrocarbon having 4 or more carbon atoms or an aromatic hydrocarbon as a raw material; producing phthalic acid by oxidizing o-xylene; or producing butadiene by oxidizing and dehydrogenating butene.

On the other hand, the present inventors diligently made investigations in order to accomplish the second object. They directed attention to the relationship between the angle of repose of a particulate packing material to be packed and the intervals between packing-material charging sites arranged in the axial direction for heat-transfer tubes. A second aspect of the disclosure has been thus completed.

More specifically, the intervals between charging sites from which a packing material is to be charged into the space between heat-transfer plates of a plate-type reactor depend on the flowability of the packing material to be packed. When the flowability of a packing material is expressed in terms of the angle of repose of the packing material, the intervals between packing-material charging sites are related to the angle of repose of the packing material to be packed.

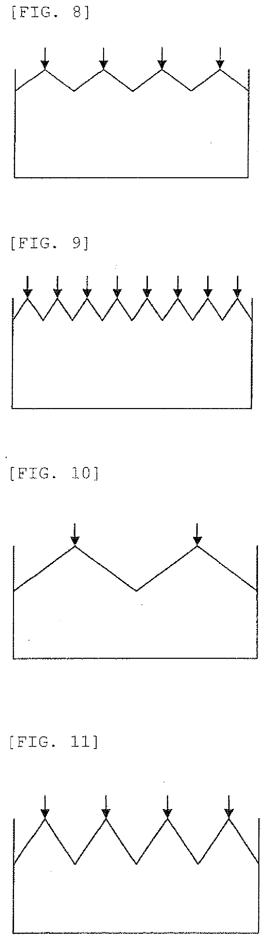

For example, as shown in Fig. 8, when a packing material having a small angle of repose is charged into the space between heat-transfer plates from charging sites arranged at appropriate intervals, the packing material is evenly packed. Furthermore, when a packing material having a large angle of repose is charged into the space between heat-transfer plates from charging sites arranged at relatively narrow intervals, the packing material is evenly packed in this case also, as shown in Fig. 9. On the other hand, in case where the intervals between packing-material charging sites are excessively widened, the packing material is unevenly packed even when having a small angle of repose, as shown in Fig. 10. Furthermore, in the case of a packing material having a large angle of repose, this packing material is unevenly packed even when the intervals between packing-material charging sites are not so widened, as shown in Fig. 11. In Fig. 8 to Fig. 11, the arrows indicate charging sites from which the packing material is charged into the space.

Accordingly, in the second aspect, when a packing material is packed into the space between heat-transfer plates of a plate-type reactor, the packing material is charged into the space from specific sites which, based on the flowability of the packing material in terms of the angle of repose, enable the packing material to form a sufficiently even packing layer.

(9) Namely, in the second aspect, is provided a method of packing a packing material into a plate-type reactor which includes a reaction vessel for reacting a raw material therein and a plurality of heat-transfer plates disposed side by side within the reaction vessel, the heat-transfer plates each including a plurality of heat-transfer tubes connected to each other at the periphery or edges of the sectional shape, the heat-transfer plates having been arranged in the reaction vessel so that the heat-transfer tubes are connected in the vertical direction, and in which a particulate packing material is charged into the space between adjacent heat-transfer plates to form a packing layer in the space, the method comprising charging the packing material into the space so that when the packing material is packed into the space from one charging site and the section where a packing layer is thus formed in the space is referred to as a packing section, then the following expression (1) is satisfied, in which $\theta$ (°) or (degree) is the angle of repose of the packing material, B (m) is the distance from the charging site for the packing material to an end of the packing section, in terms of distance in the axial direction for the heat-transfer tubes, and E (m) is 10% of a set value of the height of a highest point of the packing layer.

$$B \le E/\tan\theta \qquad (1)$$

(10) It is provided the method of packing which preferably is a method of packing the packing material into the one packing section from a plurality of sites arranged in the axial direction for the heat-transfer tubes, wherein the packing material is charged into the space so as to further satisfy the following expression (2), in which A (m) is the distance between adjacent two of the charging sites.

$$A \le 2 \times E/\tan\theta \qquad (2)$$

(11) It is provided the method of packing wherein the plate-type reactor preferably further has one or more partitions which have been disposed in the space so as to extend in the vertical direction and which separate the space into sections in the vertical direction, the packing section being each of the sections formed by the partitions.

(12) It is provided the method of packing wherein the packing section preferably has a length of from 0.05 m to 2 m in terms of length in the axial direction for the heat-transfer tubes.

(13) It is provided the method of packing wherein the packing material preferably is either a particulate catalyst or inert particles or is both the catalyst and the particles.

(14) It is preferably provided a process for product production which is a process for producing at least one reaction product from at least one raw material through a catalytic reaction using a plate-type reactor including a reaction vessel for reacting the raw material therein and a plurality of heat-transfer plates disposed side by side within the reaction vessel, the heat-transfer plates each including a plurality of heat-transfer tubes connected to each other in the vertical direction at the periphery or edges of the sectional shape, the process comprising a step in which the raw material is passed through a catalyst layer formed by dropping a catalyst into the space between adjacent heat-transfer plates and a step in which a heat medium having a desired temperature is supplied to the heat-transfer tubes, wherein the raw material is: ethylene; at least one member selected from the group consisting of hydrocarbons having 3 to 4 carbon atoms and tertiary butanol or at least one member selected from the group consisting of unsaturated aliphatic aldehydes having 3 to 4 carbon atoms; at least one aliphatic hydrocarbon having 4 or more carbon atoms; o-xylene; at least one olefin; at least one carbonyl compound; cumene hydroperoxide; butene; or ethylbenzene, and the reaction product to be obtained is: ethylene oxide; either at least one unsaturated aliphatic aldehyde having 3 to 4 carbon atoms or at least one unsaturated fatty acid having 3 to 4 carbon atoms or both the aldehyde and the acid; maleic acid; phthalic acid; at least one paraffin; at least one alcohol; acetone and phenol; butadiene; or styrene, the plate-type reactor of the invention being used for the plate-type reactor.

(15) It is provided the process for reaction-product production which preferably is a process wherein the raw material is propylene or isobutylene, and the propylene or isobutylene is oxidized with a gas containing molecular oxygen to produce either (meth)acrolein or (meth)acrylic acid or both of the two.

ADVANTAGES OF THE INVENTION

According to the first aspect of the invention, even when a raw-material gas is introduced in a large amount into a reactor in order to efficiently yield a product, this does not result in a decrease in product yield. Furthermore, the reactor has a low differential pressure and, hence, the inlet reaction pressure of the reactor can be reduced. This lowers the discharge pressure of the compressor and eliminates the necessity of high compression. Because of this, the load to be imposed on the compressor for the raw-material gas decreases and energy saving can hence be attained.

In general, the power of a compressor for a gas is governed by gas amount and gas compression ratio (compressor outlet pressure/inlet pressure). When the inlet reaction pressure or the differential pressure of the catalyst layer increases, a higher compression ratio is necessary and a higher power is required for the compressor.

In the second aspect, the packing material is charged into the space so as to satisfy expression (1). Consequently, when a packing material is packed into a plate-type reactor, the packing material can be evenly packed into the space between heat-transfer plates.

Furthermore, in the second aspect, when the packing material is packed into the one packing section from a plurality of sites arranged in the axial direction for the heat-transfer tubes, the packing material may be charged into the space so that expression (2) also is satisfied. Consequently, even when a packing material is packed into a plate-type reactor of the kind in which a packing material is packed into one packing section from a plurality of charging sites, the packing material can be evenly packed into the space between heat-transfer plates.

In the second aspect, the plate-type reactor may further have one or more partitions which have been disposed in the space so as to extend in the vertical direction and which separate the space into sections in the vertical direction, the packing section being each of the sections formed by the partitions. This configuration is more effective from the standpoint of evenly and easily packing a packing material into the space between heat-transfer plates.

Moreover, in the second aspect, the packing section may have a length of from 0.05 m to 2 m in terms of length in the axial direction for the heat-transfer tubes. This configuration is more effective from the standpoint of evenly and easily packing a packing material into the space between heat-transfer plates.

In the second aspect, the packing material may be either a particulate catalyst or inert particles or be both the catalyst and the particles. This constitution is more effective from the standpoint of conducting a gas-phase catalytic reaction with an even packing layer.

BRIEF DESCRIPTION OF THE DRAWINGS

[Fig. 1] Fig. 1 is a vertical sectional view of heat-transfer plates to be disposed in a plate-type reactor according to the first aspect of the invention.
[Fig. 2] Fig. 2 is an enlarged view of a heat-transfer plate formed by bonding two corrugated sheets.
[Fig. 3] Fig. 3 is an enlarged view of the part III of Fig. 1.
[Fig. 4] Fig. 4 is an enlarged view of the part IV of Fig. 1.
[Fig. 5] Fig. 5 is an enlarged view of the part V of Fig. 1.
[Fig. 6] Fig. 6 is a view diagrammatically illustrating the configuration of the reactor used in Examples according to the invention.
[Fig. 7] Fig. 7 is a view illustrating the spacing between the heat-transfer plates employed in the plate-type reactor of Fig. 6 and the diameter of the heat-transfer tubes.
[Fig. 8] Fig. 8 is a diagrammatic view of one example of packing layers formed by packing a packing material, from suitably spaced sites, into the space between heat-transfer plates disposed in a plate-type reactor, the packing layer being viewed from a heat-transfer plate side.
[Fig. 9] Fig. 9 is a diagrammatic view of another example of packing layers formed by packing a packing material, from suitably spaced sites, into the space between heat-transfer plates disposed in a plate-type reactor, the packing layer being viewed from a heat-transfer plate side.
[Fig. 10] Fig. 10 is a diagrammatic view of one example of packing layers formed by packing a packing material, from unsuitably spaced sites, into the space between heat-transfer plates disposed in a plate-type reactor, the packing layer being viewed from a heat-transfer plate side.
[Fig. 11] Fig. 11 is a diagrammatic view of another example of packing layers formed by packing a packing material, from unsuitably spaced sites, into the space between heat-transfer plates disposed in a plate-type reactor, the packing layer being viewed from a heat-transfer plate side.

[Fig. 12] Fig. 12 is a view diagrammatically illustrating an important part of one example of plate-type reactors according to the second aspect of the invention.

[Fig. 13] Fig. 13 is a view illustrating the spacing between the heat-transfer plates employed in the plate-type reactor of Fig. 12 and the diameter of the heat-transfer tubes.

[Fig. 14] Fig. 14 is a view diagrammatically illustrating the configuration of one embodiment of the plate-type reactor according to the second aspect.

[Fig. 15] Fig. 15 is a view illustrating a section obtained by cutting the plate-type reactor of Fig. 14 along the line A-A'.

[Fig. 16] Fig. 16 is a view illustrating a section obtained by cutting the plate-type reactor, of Fig. 14 along the line B-B'.

[Fig. 17] Fig. 17 is a view diagrammatically illustrating the configuration of a heat-medium mixer employed in the plate-type reactor of Fig. 14.

[Fig. 18] Fig. 18 is a view illustrating a partition 7 employed in the plate-type reactor of Fig. 14.

[Fig. 19] Fig. 19 is a slant view of a gas-permeable plug 8 employed in the plate-type reactor of Fig. 14.

[Fig. 20] Fig. 20 is a view illustrating the state of a gas-permeable plug 8 locked to a partition 7.

[Fig. 21] Fig. 21 is a view diagrammatically illustrating an example of packing devices which are usable in the second aspect.

[Fig. 22] Fig. 22 is a view diagrammatically illustrating another example of packing devices which are usable in the second aspect.

[Fig. 23] Fig. 23 is a view diagrammatically illustrating an example of packing apparatus usable in the second aspect.

[Fig. 24] Fig. 24 is a view diagrammatically illustrating another example of packing apparatus usable in the second aspect.

[Fig. 25] Fig. 25 is a view diagrammatically illustrating a top area of a packing layer formed by charging a packing material into the space between heat-transfer plates from a plurality of charging sites.

[Fig. 26] Fig. 26 is a view diagrammatically illustrating one example of distributors usable in the second aspect.

## MODES FOR CARRYING OUT THE INVENTION

### <First Aspect of the Invention>

In the method of reaction of the invention as defined in the claims, use is made of a reactor which is equipped with a catalyst layer for reacting a raw-material gas fed and is capable of cooling and heating through heat exchange with the catalyst layer. In the invention, the reactor is a plate-type reactor which is equipped with a catalyst layer formed between adjacent heat-transfer plates and in which a raw-material gas fed passes through the space between the adjacent heat-transfer plates and is then discharged.

A shell-and-tube reactor is disclosed for reference.

Examples of shell-and-tube reactors include fixed-bed multitubular heat-exchange-type reactors such as that described in, e.g., JP-A-2003-267912. Preferred examples of the plate-type reactor include a reactor which has heat-transfer plates each formed by disposing, face to face, two corrugated sheets having a shape including a circular arc, an elliptic arc, or part of a rectangle or polygon and bonding the ridges of one of the corrugated sheets to the ridges of the other to form a plurality of heat-medium passages and in which the heat-transfer plates have been arranged so that the corrugated-sheet ridges of one of any adjacent heat-transfer plates face the corrugated-sheet grooves of the other to form a catalyst layer having a given thickness.

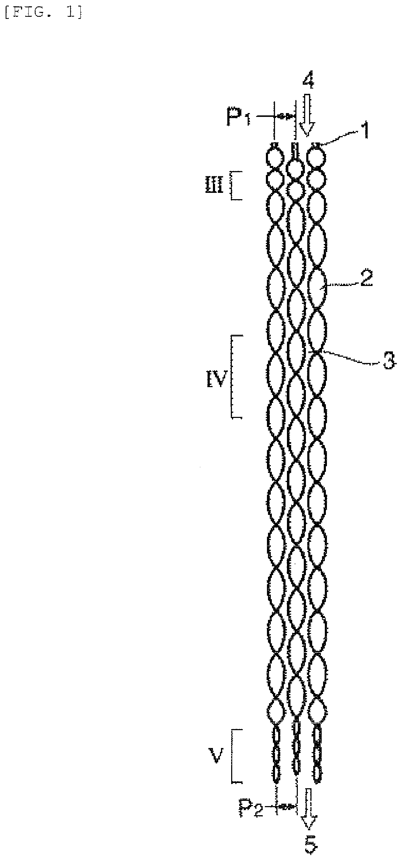

An example of plate-type reactors applicable to the invention is described by reference to Fig. 1 to Fig. 5.

In Fig. 1, each heat-transfer plate 1 is constituted of two corrugated sheets disposed face to face. The heat-transfer plate 1 has a plurality of heat-medium passages 2 formed inside the two corrugated sheets. The space 3 between adjacent heat-transfer plates 1 can be packed with a catalyst, and packing a catalyst thereinto gives a catalyst layer. A raw-material reactant gas is fed through a reactant-gas inlet 4, passes through the catalyst layer, and is discharged from the plate-type reactor through a reactant-gas outlet 5 after a target product is yielded by a reaction. Although the direction in which the raw-material reactant gas flows is not limited, a downward flow or an upward flow is usually employed.

A heat medium is supplied to the multiple heat-medium passages 2 formed within each heat-transfer plate 1, and is caused to flow in a cross-flow direction with respect to the direction of flow of the raw-material reactant gas. Through the heat-transfer plates 1, the heat medium supplied cools the catalyst layers in the case of an exothermic reaction or heats the catalyst layers in the case of an endothermic reaction. Thereafter, the heat medium is discharged from the plate-type reactor.

The shape of each heat-transfer plate is determined according to the shape and size of the reaction vessel. However, the shape thereof is generally rectangular. The size of each heat-transfer plate is determined according to the shape and size of the reaction vessel. However, in the case of rectangular heat-transfer plates, for example, the vertical dimension thereof (i.e., the connection height of the heat-transfer tubes) may be 0.5-10 m and is preferably 0.5-5 m, more preferably 0.5-3 m. In view of the size of generally available steel sheets, two plates may be bonded to each other or used in combination when a vertical dimension of 1.5 m or larger is to be obtained. The horizontal dimension thereof (i.e., the length of the heat-transfer tubes) is not particularly limited, and is generally 0.1-20 m. The horizontal dimension thereof is preferably 3-15 m, most preferably 6-10 m. The number of heat-transfer plates is determined according to the amount of the catalyst to be used in the reaction. However, the number thereof is generally 10-300.

In the reaction vessel, adjacent heat-transfer plates may be arranged so that the surface ridges of one of the heat-transfer plates face those of the other, or may be arranged so that the surface ridges of one heat-transfer plate face the surface grooves of the other heat-transfer plate. In Fig. 1, adjacent heat-transfer plates have been arranged so that the surface ridges of one heat-transfer plate face the surface grooves of the other heat-transfer plate. It is preferred that the distance between adjacent heat-transfer plates should be regulated so as to be in the range of 23-50 mm in terms of the average distance between the heat-transfer-tube major axes of the respective heat-transfer plates (1.1-5 times the sum of half of the width of the heat-transfer tubes of one of the adjacent heat-transfer plates and half of the width of the heat-transfer tubes of the other) so that a space having a width of about 3-40 mm, in terms of width in the transverse direction for the heat-transfer tubes, is formed between the heat-transfer plates.

From the standpoint of controlling the reaction of a raw material by regulating the temperature of the heat medium to be passed through the heat-transfer tubes, it is preferred that the heat-transfer tubes in each heat-transfer plate should have been formed so as to extend in the direction perpendicular to the direction of gas passing in the reaction vessel. Namely, it is preferred that the direction in which the heat medium flows through the heat-transfer tubes should be perpendicular to the direction of gas passing in the reaction vessel.

The heat-transfer tubes are constituted of a material having such thermal conductivity that heat exchange occurs between the heat medium present in the heat-transfer tubes and the catalyst layers in contact with the outer surfaces of the heat-transfer tubes. Examples of such a material include stainless steels, carbon steel, Hastelloy, titanium, aluminum, engineering plastics, and copper. It is preferred to use a stainless steel. Preferred of the stainless steels are 304, 304L, 316, and 316L. The sectional shape of the heat-transfer tubes may be circular or may be an approximately circular shape such as, e.g., an elliptic shape or a Rugby ball shape. The sectional shape thereof may also be a polygonal shape, e.g., rectangle. The term "periphery of the sectional shape of a heat-transfer tube" means the periphery of a circle. The term "edges of the sectional shape of a heat-transfer tube" means the major-axis ends of an approximately circular shape or corner edges of a polygonal shape.

In each heat-transfer plate, the multiple heat-transfer tubes may have the same sectional shape and size or may differ in sectional shape or size. With respect to the size of the sectional shape of each heat-transfer tube, the width of the heat-transfer tube is, for example, 5-50 mm and the height of the heat-transfer tube is, for example, 10-100 mm.

The plate-type reactor can be configured so as to include one or more catalyst layers having a single average catalyst-layer thickness. In the invention, each catalyst layer is divided into a plurality of reaction zones differing in average catalyst-layer thickness. The term "average catalyst-layer thickness" herein means the average distance between those surfaces of adjacent heat-transfer plates between which the catalyst layer is sandwiched, as measured in the direction perpendicular to the flow of a raw-material gas. The multiple reaction zones can be independently supplied with a heat medium. In the case of an exothermic reaction, for example, the heat generated by the reaction is removed through the heat-transfer plates, whereby the temperatures of the catalyst layer can be independently controlled.

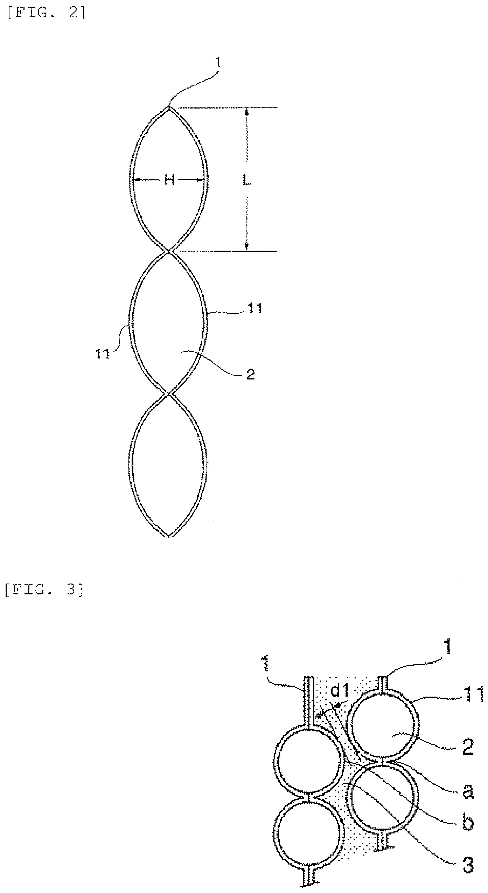

The configuration of the heat-transfer plates 1 is explained in greater detail by reference to Fig. 2 to Fig. 5.

The heat-transfer plate 1 shown in Fig. 2 is formed by bonding two corrugated sheets 11 to each other. In Fig. 2, the corrugations are constituted of part of arcs. However, the shape thereof is not particularly limited, and can be determined while taking account of ease of production and the flow of a raw-material reactant gas. The height of the corrugations (H) and the period of the corrugations (L) also are not particularly limited. However, the height (H) thereof is preferably 5-50 mm, more preferably 10-30 mm. The period (L) thereof is preferably 10-100 mm, more preferably 20-50 mm. The height and period thereof are determined while taking account of the heat accompanying the reaction to be conducted in the catalyst layer and the flow rate of the heat medium for removing the heat or heating the catalyst layer. With respect to the spacing (P) between two heat-transfer plates, the distance between the heat-transfer-tube major axes of the respective heat-transfer plates is regulated to 10-50 mm, preferably 20-35 mm, provided that the distance is in the range of 1.1-2 times the sum of half of the width of the heat-transfer tubes of one of the adjacent heat-transfer plates and half of the width of the heat-transfer tubes of the other, so that a space having a width of 3-40 mm, in terms of width in the transverse direction for the heat-transfer tubes, is formed between the surfaces of the heat-transfer plates.

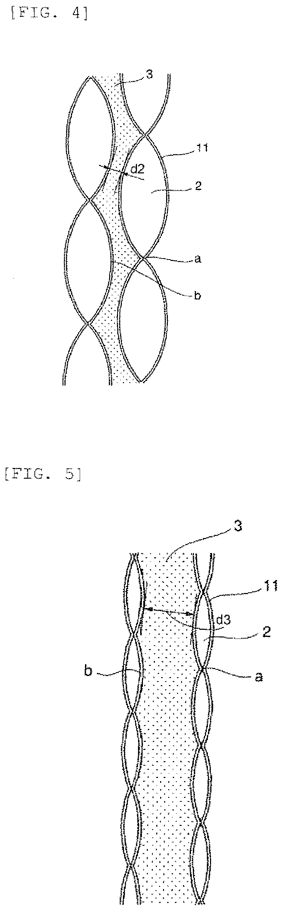

Fig. 3 to Fig. 5 [Fig. 3 is an enlarged view of the part III of Fig. 1; Fig. 4 is an enlarged view of the part IV of Fig. 1; and Fig. 5 is an enlarged view of the part V of Fig. 1] illustrate the shapes of heat-transfer plates 1 located near the raw-material-reactant-gas inlet, in an intermediate area, and near the raw-material-reactant-gas outlet, respectively.

Each heat-transfer plate 1 has been formed by disposing, face to face, two corrugated sheets 11 having a shape including a circular arc, elliptic arc, or rectangle and bonding the ridges a of one of the corrugated sheets 11 to the ridges a of the other to form a plurality of heat-medium passages 2. Adjacent heat-transfer plates 1 are arranged so that the corrugated-sheet ridges a of one of the plates 1 face the corrugated-sheet grooves b of the other at a given distance to form a space 3.

Symbols d1, d2, and d3 in the figures denote the minimum spacing of the space 3 between the adjacent heat-transfer plates 1 in the part III, part IV, and part V, respectively. The values of d1, d2, and d3 can be changed by suitably changing the circular arc, elliptic arc, or rectangular shape imparted to the corrugated sheets 11. In Fig. 3 to Fig. 5, the minimum spacings have been regulated so that d1<d2<d3. In the plate-type reactor used according to the invention, when there are multiple values of minimum spacing (d), the term "minimum spacing (d) of the space between heat-transfer plates" means the smallest one (d) of the values of minimum spacing. For example, when there are d1, d2, and d3 as in the case shown above, that term means the smallest of the d1, d2, and d3. Namely, in Figs. 3 to 5, that term means d1 when d1<d2<d3.

The value (d) is generally regulated to about 2-50 mm. However, (d) is preferably 5-20 mm.

In Fig. 1, with respect to the spacing (P) between adjacent heat-transfer plates 1 arranged, the spacing P1 at the reactant-gas inlet 4 is the same as the spacing P2 at the reactant-gas outlet 5. Namely, in the invention the adjacent heat-trans fer plates 1 have been disposed so as to be parallel with each other. Examples of materials usable for the corrugated sheets 11 include stainless steels, carbon steel, Hastelloy, titanium, aluminum, engineering plastics, and copper. It is preferred to use a stainless steel. Preferred of the stainless steels are 304, 304L, 316, and 316L. The thickness of each corrugated sheet 11 may be 2 mm or smaller and is preferably 1 mm or smaller. It is preferred that one or more partitions extending vertically (i.e., in the direction of connection height of the heat-transfer tubes), that is, extending in the direction of gas passing, should be disposed in the space between the adjacent heat-transfer plates, from the standpoints of holding the catalyst packed in each section and of causing the partitions to function as a spacer for maintaining a distance between the heat-transfer plates. The intervals between the partitions to be disposed are preferably from 5 cm to 2 m, more preferably from 10 cm to 1 m, especially preferably from 20 cm to 50 cm.

Any shell-and-tube reactor or tube embodiments mentioned in the following are reference embodiments.

The method of reaction is characterized in that the tubes (in a shell-and-tube reator; reference) and heat-transfer plates (in a plate-type reactor; invention) through which a reactant gas passes are configured so as to have a shape which enables the gas to be apt to flow smoothly, whereby the pressure loss generating when the reactant gas comes into contact with the tubes and heat-transfer plates can be reduced. For example, the tubes or heat-transfer plates in the reactor are configured so as to have such a shape that the reactor, in the state of including no catalyst layer in the tubes (shell-and-tube reactor) or between the adjacent heat-transfer plates (plate-type reactor), has a differential pressure of 50 Pa or lower when air having ordinary temperature is introduced into the reactor at a space velocity, in terms of normal-state velocity per unit catalyst layer volume, of 7,200 (1/hr) while regulating the reactor outlet pressure to ordinary pressure and without conducting heating with a heat medium. As a result, a reduction in pressure loss can be attained. The differential pressure is preferably 40 Pa or lower, more preferably 30 Pa or lower.

In the method of reaction, a plate-type reactor is used which, in the state of being equipped with a catalyst layer in the tubes in the case of a shell-and-tube reactor or in the spaces between the adjacent heat-transfer plates in the case of a plate-type reactor, has a differential pressure of 15 kPa or lower when air having ordinary temperature (0°) is introduced into the reactor at a space velocity, in terms of normal-state velocity per unit volume of the catalyst layer, of 1,800 (1/hr) while regulating the reactor outlet pressure to ordinary pressure (1 atm) and without conducting heating with a heat medium.

Such state in which air having ordinary temperature (0°) is introduced into the reactor at a space velocity, in terms of normal-state velocity per unit volume of the packed space, of 1,800 (1/hr) while regulating the reactor outlet pressure to ordinary pressure (1 atm) and without conducting heating with a heat medium is intended to simulate the case where a relatively large amount of a raw-material gas has been introduced into the reactor in order to improve reaction efficiency. In conventional reactions conducted under such conditions, the introduction of a large amount of a raw-material gas into a reactor for the purpose of improving reaction efficiency has resulted in an increase in the differential pressure of the reactor to pose problems such as, e.g., a decrease in product yield. A reactor which, when operated under those conditions, has a differential pressure of 15 kPa or lower is preferred because use of this reactor enables a high yield to be maintained even under such conditions that a raw material is fed in a large amount relative to catalyst layer amount, specifically, the space velocity of the raw-material gas is 1,100 (1/hr) or higher, preferably 1,800 (1/hr) or higher. Consequently, the invention is suitable for use under such conditions that a raw-material gas is introduced into the reactor in a large amount relative to catalyst layer amount, that is, the raw-material gas is introduced at a space velocity, in terms of normal-state velocity per unit volume of the catalyst layer, of 1,100 (1/hr) or higher, preferably 1,800 (1/hr) or higher. However, it is preferred to conduct the reaction at a space velocity of 4,200 (1/hr) or lower because space velocities higher than that result in a considerable increase in differential pressure.

The differential pressure of a reactor is referred to also as pressure loss. Methods for measuring the differential pressure are not particularly limited. Examples thereof include a method in which a mass flow meter is used to cause a gas to flow through a reaction tube at a given rate and the resultant pressures are measured. Differential pressure can be determined, for example, with an existing manometer or differential manometer or by a method in which the areas where the pressure is to be measured are connected with a tube containing water and the differential pressure is determined from the level difference between the water columns. The term "normal state" means the conditions of 0°C and 1 atm.

From the standpoint of further improving the yield of a reaction product, the differential pressure is preferably 12 kPa or lower, more preferably 10 kPa or lower.

The following techniques are thought to be used for attaining the differential pressure of the reactor.

(1) To reduce the length of the reactor as measured in the raw-material gas flow direction.

The reactor length in the flow direction for a raw-material gas is reduced and the distance over which the raw-material gas flows is shortened, whereby a reduction in differential pressure can be attained. In the case of a plate-type reactor, the length thereof in the raw-material gas flow direction is generally about 0.5-10 m and is preferably 0.5-5 m, more preferably 0.5-3 m.

(2) To employ tubes and heat-transfer plates which have low surface roughness.

The tubes and heat-transfer plates through which a reactant gas passes are constituted of ones having a reduced surface roughness, whereby the pressure loss generating when the reactant gas comes into contact with the tubes and heat-transfer plates can be reduced. The maximum surface roughness (Rmax) of the tubes and heat-transfer plates according to the invention is generally 12.5 s or lower, preferably 8 s or lower, more preferably 3.2 s or lower.

(3) To impart to heat-transfer plates a shape which facilitate gas flow.

The heat-transfer plates through which a reactant gas flows are configured so as to have a shape which enables the gas to be apt to flow smoothly. Thus, the pressure loss generating when the reactant gas comes into contact with the heat-transfer plates can be reduced.

(4) To change the porosity of catalyst layers.

When a catalyst layer is packed into each tube or into the space between adjacent heat-transfer plates, the catalyst is disposed so that the porosity of the catalyst layer deceases from the raw-material gas inlet toward the raw-material gas outlet. Namely, the amount of interstices within the catalyst layer in an area near the raw-material gas inlet, where the gas reacts in a large amount, is regulated to a relatively large value, while the amount of interstices within the catalyst layer in an area near the raw-material gas outlet, where the gas reacts in a smaller amount, is regulated to a small value. Thus, the reaction can be controlled and a relatively small pressure loss can be obtained.

The term "porosity of the catalyst layer" means the proportion of that part of the catalyst-packed space which is not occupied by the catalyst. For example, the porosity can be determined by calculating the volume of the whole charged catalyst from the weight of the charged catalyst, catalyst weight per particle, and catalyst volume per particle and calculating the proportion of the space not occupied by the catalyst to the volume of the catalyst layer between the heat-transfer plates. The term "catalyst volume" means the volume of the catalyst including internal pores.

The catalyst layer is divided into a plurality of reaction zones arranged from the raw-material gas inlet toward the raw-material gas outlet as defined in the claims. For example, the catalyst layer can be divided into a reaction zone, as a first reaction zone, located nearest to the raw-material gas inlet and a second reaction zone and a third reaction zone which are arranged in this order toward the raw-material gas outlet. In the case where the catalyst layer has been divided into these reaction zones, the porosity is regulated to be highest in the first reaction zone and to decrease therefrom to the second reaction zone and to the third reaction zone, whereby the differential pressure can be reduced. The porosity of the catalyst layer in the invention is preferably regulated to 50% or higher in the first reaction zone and to 50% or lower in the second and succeeding reaction zones, and is more preferably regulated to 50-65% in the first reaction zone and to 40-50% in the second and succeeding reaction zones.

Furthermore, the difference in porosity between the first reaction zone and the second reaction zone is regulated to preferably 3% or larger, more preferably 5% or larger, even more preferably 10% or larger.

Examples of methods for disposing the catalyst layer used in the invention so that the porosity of the catalyst layer decreases from the raw-material gas inlet toward the raw-material gas outlet include to use catalysts which satisfy the following requirements.

(a) A catalyst is packed so that when the catalyst layer is divided into a plurality of reaction zones, the diameter (D) of the catalyst being packed decreases from the reactant-gas inlet toward the reactant-gas outlet. Alternatively, catalysts differing in particle diameter may be mixed together, whereby the porosity can be regulated.

(b) A catalyst is packed so that when the catalyst layer is divided into a plurality of reaction zones, the bulk density of the catalyst being packed increases from the reactant-gas inlet toward the reactant-gas outlet.

(c) In the invention, a catalyst layer is divided into a plurality of reaction zones so that the thickness of the catalyst layer being packed, in terms of thickness in the direction of the length of the space between the adjacent heat-transfer plates, increases from the reactant-gas inlet toward the reactant-gas outlet. In a shell-and-tube reactor, the inner diameter of the tubes is preferably increased continuously or stepwise from the reactant-gas inlet toward the reactant-gas outlet to thereby reduce the porosity of the catalyst layer.

(d) In the invention, the ratio of the particle diameter (D) of the catalyst to be packed into the catalyst layer to the minimum spacing (d) between the heat-transfer plates in the plate-type reactor according to the invention (D/d) is regulated so as to be 0.9>D/d>0.1.

(e) The catalyst to be packed into the reaction zones is mixed with a diluent having no catalytic activity to suitably regulate the porosity of the catalyst layer.

Preferred examples of the shape of the catalyst to be used in the invention include spherical shapes having a diameter of 1-15 millimeters (mm), pellet shapes having a major-axis length of 2-15 mm, cylindrical shapes having an outer diameter of 1-15 mm and a height of 2-15 mm, and cylindrical ring shapes having a hole at the cylinder center which have an outer diameter of 3-15 mm, inner diameter of 1-5 mm, and height of 2-10 mm.

Other preferred examples of the shape of the catalyst to be used in the invention include disk shapes having a thickness of 2-4 mm and a diameter of 2-30 mm, platy shapes which have a thickness of 2-4 mm and in which the dimension of the longest distance between two points on the periphery of a section formed by cutting the platy shape perpendicularly to the thickness direction is 2-30 mm, and rod shapes which have an axial-direction length of 2-30 mm and in which the dimension of the longest distance between two points on the periphery of a section formed by cutting the rod shape perpendicularly to the axial direction (diameter in the case of a circular section) is 1-4 mm.

The term "particle diameter (D) of a catalyst" used in the invention has the following meanings. When the catalyst has the spherical shape, the diameter thereof is D. When the catalyst has the pellet shape, D is the major-axis length. When the catalyst has the cylindrical or ring shape, D is the larger of the outer diameter of the circle and the height. When the catalyst has the disk shape, D is the outer diameter of the circle. When the catalyst has the platy shape, D is the dimension of the longest distance between two points on the periphery of a section formed by cutting the shape perpendicularly to the thickness direction. When the catalyst has the rod shape, D is the axial-direction length.

The term "major-axis length of the pellet shape" means the distance between two parallel planes between which the pellet is sandwiched so that the distance therebetween is maximum among the values corresponding to pellet orientation at all angles.

With respect to the bulk density of the catalyst to be used in the invention, preferred examples include catalysts having a bulk density of 0.4-2.0 kg/L, more preferably 0.6-1.6 kg/L. Bulk density in the invention means the value determined by packing the catalyst into a vessel having a capacity of, for example, 1 L, measuring the mass of this catalyst, and dividing the mass by the volume.

The bulk-state porosity of the catalyst to be used in the invention is, for example, preferably 60% or lower, more preferably 50% or lower, even more preferably 45% or lower, especially preferably 40% or lower. The term "bulk-state porosity" of the catalyst in the invention means the value obtained by the following method. The catalyst filling a vessel having a capacity at least 10,000 times the volume of one catalyst particle, for example, in the case of a catalyst having a volume per particle of 0.1 cc, a vessel having a capacity of 1 L, i.e., 10,000 times the particle volume, is weighed, and the volume occupied by the catalyst is subtracted from 1 L. The proportion in % of the volume of the vacant space where the catalyst is not present is taken as the porosity. The volume of the catalyst in this porosity determination includes the volume of internal pores.

Porosity varies depending mainly on catalyst shape and catalyst particle diameter. For example, ring-form catalysts having an outer diameter of 5 mm, inner diameter of 2 mm, and height of 3 mm have a porosity of from 60% to 50%. Disk-form catalysts having an outer diameter of 4 mm and a height of 3 mm and spherical catalysts having a diameter of 5 mm have a porosity of from 50% to 35%. Even in catalysts which all are in a disk form, the precise value varies depending on whether the surface has roughness or not and on whether the edges are rounded or not.

With respect to the influence of particle diameter, particles which are not truly spherical and which have a small particle diameter or a particle diameter distribution have a reduced porosity.

Porosity is determined, for example, through calculation from bulk density and the specific gravity of the catalyst particles. However, there is no strictly fixed method for measuring, in particular, the bulk density of catalyst particles, and slightly fluctuated values are obtained depending on measuring methods (constant-volume method, constant-weight method, whether tapping is conducted or not, packing height in vessel, etc.) and on the shape of the volume-measuring vessel, e.g., 1-L vessel.

In the case (a) where a catalyst is packed so that when the catalyst layer is divided into a plurality of reaction zones, the diameter (D) of the catalyst being packed decreases from the reactant-gas inlet toward the reactant-gas outlet, it is preferred that (D) in the first reaction zone should be 5-15 mm and (D) in the second and succeeding reaction zones should be a value which is smaller than in the first reaction zone and is 3-10 mm. It is more preferred that (D) in the first reaction zone should be 7-12 mm and (D) in the second and succeeding reaction zones should be a value which is smaller than in the first reaction zone and is 4-7 mm.

The difference in particle diameter (D) between the first reaction zone and the second reaction zone is preferably 1 mm or larger, more preferably 2 mm or larger.

In the case (b) where a catalyst is packed so that when the catalyst layer is divided into a plurality of reaction zones, the bulk density of the catalyst being packed increases from the reactant-gas inlet toward the reactant-gas outlet, it is preferred that the bulk density thereof in the first reaction zone should be 0.6-1.4 kg/L and the bulk density thereof in the second and succeeding reaction zones should be a value which is larger than in the first reaction zone and is 0.8-1.6 kg/L. It is more preferred that the bulk density thereof in the first reaction zone should be 0.7-1.2 kg/L and the bulk density thereof in the second and succeeding reaction zones should be a value which is larger than in the first reaction zone and is 0.8-1.4 kg/L.

The difference in bulk density between the first reaction zone and the second reaction zone is preferably 0.05 kg/L or larger, more preferably 0.1 kg/L or larger.

In the inventive case (c) where a catalyst layer is divided into a plurality of reaction zones so that the thickness of the catalyst layer being packed, in terms of thickness in the direction of the length of the space between the adjacent heat-transfer plates, increases from the reactant-gas inlet toward the reactant-gas outlet, it is preferred that the thickness of the catalyst layer in the first reaction zone should be 5-20 mm and the thickness thereof in the second and succeeding reaction zones should be a value which is larger than in the first reaction zone and is 10-30 mm. It is more preferred that the thickness thereof in the first reaction zone should be 7-10 mm and the thickness thereof in the second and succeeding reaction zones should be a value which is larger than in the first reaction zone and is 10-16 mm. In the invention, the term "direction of the length of the space between adjacent heat-transfer plates" means the direction which is perpendicular to the flow direction for a raw-material gas and in which the distance between the adjacent heat-transfer plates is minimum. The term "thickness of the catalyst layer" means not the maximum or minimum value of catalyst-layer thicknesses as measured in the direction of the length of the space between heat-transfer plates in each reaction zone but the average of catalyst-layer thicknesses as measured in the direction of the length of the space between heat-transfer plates in the reaction zone.

The difference in the thickness of the catalyst layer between the first reaction zone and the second reaction zone is preferably 1 mm or larger, more preferably 2 mm or larger.

Furthermore, in the invention, it is also possible to reduce pressure loss by (d) regulating the ratio of the particle diameter (D) of the catalyst to be packed into the catalyst layer to the minimum spacing (d) between the heat-transfer plates in the plate-type reactor according to the invention (D/d) to a value satisfying $0.9 > D/d > 0.1$. The value of (D/d) preferably satisfied $0.9 > D/d > 0.3$, more preferably satisfies $0.7 > D/d > 0.5$.

When there are multiple values of minimum spacing (d) (when there are d1, d2, and d3 as shown above) in one plate-type reactor, the ratio (D/d) means the ratio of the particle diameter (D) of the catalyst present in the area having the smallest one of the values of minimum spacing to the smallest spacing (d) of those values of minimum spacing. In case where (D/d) is 0.1 or smaller, the catalyst packed into the space between the heat-transfer plates has too small a particle diameter (D) and this causes an excessive pressure loss when a raw-material reactant gas is passed through. On the other hand, in case where (D/d) is 0.90 or larger, the arch formation called bridging tends to occur. In case where bridging has occurred, the reaction does not proceed in an expected way because the catalyst has not been packed in a given amount, or there is the trouble of, for example, discharging the catalyst and re-packing the catalyst.

Methods for catalyst packing

(hereinafter also referred to simply as packing methods) are not particularly limited so long as the catalyst can be packed so as to satisfy the requirements described above. The catalyst may be randomly packed into layers to be filled, or may be packed into regularly arranged layers. However, it is preferred to pack the catalyst randomly, from the standpoint of ease of catalyst packing. Preferred examples of methods for random packing include a method in which a catalyst-packing device equipped with a conveying member is used to pack a catalyst into the space between adjacent heat-transfer plates from above the heat-transfer plates and which is characterized in that the particles of the catalyst are not vertically stacked up at the end of the conveying member. The term "particles of the catalyst are not vertically stacked up" means that the catalyst at the end of the conveying member is in a single-layer state.

The catalyst-packing device equipped with a conveying member, use of which is preferred, is not particularly limited so long as the packing device can be regulated so that catalyst particles are not vertically stacked up at the end of the conveying member.

The method of reaction can be applied, without particular limitations, to any shell-and-tube reactor (reference) or plate-type reactor (invention) for gas-phase catalytic oxidation reactions. Examples of processes utilizing such a gas-phase catalytic oxidation reaction include: a process in which ethylene is oxidized to produce ethylene oxide; and a process in which at least one raw organic-compound gas selected from the group consisting of hydrocarbons having 3 to 4 carbon atoms and tertiary butanol or at least one raw organic-compound gas selected from the group consisting of unsaturated aliphatic aldehydes having 3 to 4 carbon atoms is fed together with a raw-material reactant gas containing molecular oxygen to subject the raw organic-compound gas to a gas-phase catalytic oxidation reaction and thereby produce one or more reaction products selected from the group consisting of unsaturated aliphatic aldehydes having 3 to 4 carbon atoms and unsaturated fatty acids having 3 to 4 carbon atoms. Examples of the reaction products include (meth)acrolein, (meth)acrylic acid, ethylene oxide, either one or more unsaturated aliphatic aldehydes having 3 to 4 carbon atoms or one or more unsaturated fatty acids having 3 to 4 carbon atoms or both of these, maleic acid, and phthalic acid.

Of such processes, the following are preferred processes to which the method of reaction is applied: a process in which propylene or isobutylene is used as a raw material to produce (meth)acrolein and (meth)acrylic acid; a process in which (meth) acrolein is used as a raw material to produce (meth)acrylic acid; a process in which an aliphatic hydrocarbon having 4 or more carbon atoms or an aromatic hydrocarbon is used as a raw material to produce maleic acid; and a process in which butene is oxidized and dehydrogenated to produce butadiene. In particular, the process for producing (meth)acrolein and (meth)acrylic acid is highly exothermic, and it is more preferred to use the method of the invention in this process.

In the method of reaction of the invention, known catalysts can be used according to purposes. Examples thereof include metal oxides containing molybdenum, tungsten, bismuth, etc. or metal oxides containing vanadium, etc.

In the case where the raw-material gas is propylene, preferred examples of the metal oxides include compounds represented by the following general formula (1).

$$Mo_{(a)}Bi_{(b)}Co_{(c)}Ni_{(d)}Fe_{(e)}X_{(f)}Y_{(g)}Z_{(h)}Q_{(i)}Si_{(j)}O_{(k)} \qquad \text{Formula (1)}$$

In formula (1), Mo represents molybdenum; Bi represents bismuth; Co represents cobalt; Ni represents nickel; Fe represents iron; X represents at least one element selected from the group consisting of sodium, potassium, rubidium, cesium, and thallium; Y represents at least one element selected from the group consisting of boron, phosphorus, arsenic, and tungsten; Z represents at least one element selected from the group consisting of magnesium, calcium, zinc, cerium, and samarium; Q represents a halogen element; Si represents silicon; and O represents oxygen.

Symbols a, b, c, d, e, f, g, h, i, j, and k represents the atomic proportions of the Mo, Bi, Co, Ni, Fe, X, Y, Z, Q, Si, and O, respectively. When the proportion of molybdenum atoms (Mo) is 12, the other symbols are: $0.5 \leq b \leq 7$, $0.5 \leq c \leq 10$, $0 \leq d \leq 10$, $1 \leq c+d \leq 10$, $0.05 \leq e \leq 3$, $0.0005 \leq f \leq 3$, $0 \leq g \leq 3$, $0 \leq h \leq 1$, $0 \leq h \leq 1$, and $0 \leq j \leq 40$; and k is a number determined by the oxidized state of each element.

On the other hand, in the case where the raw organic-compound gas is (meth)acrolein, preferred examples of the metal oxides include compounds represented by the following general formula (2).

$$Mo_{(12)}V_{(a)}X_{(b)}Cu_{(c)}Y_{(d)}Sb_{(e)}Z_{(f)}Si_{(g)}C_{(h)}O_{(i)} \qquad \text{Formula (2)}$$

In formula (2), X represents at least one element selected from the group consisting of Nb and W. Y represents at least one element selected from the group consisting of Mg, Ca, Sr, Ba, and Zn. Z represents at least one element selected from the group consisting of Fe, Co, Ni, Bi, and Al. In the formula, Mo, V, Nb, Cu, W, Sb, Mg, Ca, Sr, Ba, Zn, Fe, Co, Ni, Bi, Al, Si, C, and O are symbols of elements. Furthermore, a, b, c, d, e, f, g, h, and i represent the atomic proportions of the respective elements. Relative to the proportion of molybdenum atoms (Mo) of 12, the symbols are: $0 < a \leq 12$, $C \leq b \leq 12$, $0 \leq c \leq 12$, $0 \leq d \leq 8$, $0 \leq e \leq 500$, $0 \leq f \leq 500$, $0 \leq g \leq 500$, and $0 \leq h \leq 500$; and i is a number determined by the oxidized state of each of the elements excluding C.

The heat medium to be supplied to the shell side in the shell-and-tube reactor (reference) and the heat medium to be supplied to the heat-medium passages of the heat-transfer plates in the plate-type reactor (invention) are not particularly limited so long as reaction temperature can be controlled therewith. However, a molten salt which is a mixture of nitric acid salts (niter) and a high-boiling organic heat medium constituted of, e.g., a mixture of polycyclic aromatic hydrocarbons are preferred. With respect to the temperature of such a heat medium, it is preferred that the heat medium should be supplied at 200-600°C, more preferably at 200-500°C. In the case where the raw-material gas is propylene, it is preferred that the temperature of the heat medium to be supplied to the heat-medium passages should be 250-400°C. On the other hand, in the case where the raw-material gas is acrolein, it is preferred that the temperature of the heat medium to be supplied to the heat-medium passages should be 200-350°C.

The difference in the temperature of the heat medium between the inlet and the outlet is preferably 0.5-10°C, more preferably 2-5°C. With respect to each of the heat-medium passages, the flow rate, temperature, and flow direction of the heat medium may be changed from passage to passage or from passages to passages.

When the plate-type reactor is constituted of a plurality of reaction zones which differ in catalyst-layer thickness in terms of average layer thickness in the direction of the length of the space between the adjacent heat-transfer plates, as described in Fig. 1, then a heat medium is supplied to the multiple reaction zones respectively at optimal temperatures. There are cases where even in a single reaction zone, a heat medium is caused to flow independently through the individual passages or through sets of two or more passages at the same temperature either in the same direction or in counterflow directions. Furthermore, a heat medium which was supplied to the heat-medium passages of one reaction zone and has been discharged therefrom can be supplied to the heat-medium passages of the same or another reaction zone.

It is preferred that the temperature of the heat medium in the same reaction zone should be basically constant. It is, however, possible to change the temperature thereof in such a range that a hot-spot phenomenon does not occur.

The flow rate of the heat medium being supplied to the heat-medium passages is determined by the quantity of heat of reaction and resistance to heat transfer. However, resistance to heat transfer is less apt to be problematic because the resistance is usually possessed by the gas, i.e., the raw-material gas, rather than the heat medium, which is liquid. The linear velocity of the liquid in the heat-medium passages preferably is 0.3-2 m/s. From the standpoint of reducing the heat-medium-side resistance to heat transfer to a value which is smaller than that of heat transfer resistance on the raw organic-compound gas side and is not problematic, a linear velocity of 0.5-1 m/s is most suitable. Too large values thereof are undesirable from the standpoint of profitability because the pump for circulating the heat medium necessitates a higher power.

<Second Aspect of the Disclosure>

The method for packing a packing material into the plate-type reactor is a method which comprises charging the packing material into the space so that when the packing material is packed into the space from one charging site and the section where a packing layer is thus formed in the space is referred to as a packing section, then the following expression (1) is satisfied, in which θ (degree)is the angle of repose of the packing material, B (m) is the distance from the charging site for the packing material to an end of the packing section, in terms of distance in the axial direction for the heat-transfer tubes, and E (m) is 10% of a set value of the height of a highest point of the packing layer.

$$B \leq E/\tan\theta \qquad\qquad (1)$$

The charging site is that side edge of the packing-material flow being charged into the space which is on the side facing that end. In the case where the width of the flow of the packing material is 0.05 m or smaller, the center of the flow of the packing material may be taken as the charging site. When the width of the flow of the packing material is small, the operation of charging the packing material is apt to be easy. When the width thereof is large, the evenness of the packing material charged is apt to be heightened. From the standpoint of the efficiency of charging operation, the width of the flow of the packing material is preferably 0.05 m or smaller. From the standpoint of improving the evenness of the packing material charged, the width of the flow of the packing material is preferably 0.1 m or larger, more preferably 0.15 m or larger.

The term "highest point of the packing layer" means the highest point of the top of the packing material packed in one packing section. When the packing material is charged from one charging site per packing section, the height of a highest point of the packing layer is the height of the packing layer as measured at the charging site. When the packing material is charged from a plurality of charging sites per packing section, the height of a highest point of the packing layer is the largest value of the heights of the packing layer as measured respectively at the charging, sites. Herein, a set value of the height of the packing layer is given in terms of the height of a highest point of the packing layer. For example, in the invention, when the set value of the height of the packing layer is 1.7 m, this means that the height of a highest point of the packing layer is set at 1.7 m. It is preferred that a packing material should be packed so that the difference between a set value of the height of a highest point of the packing layer and the found value of the height of a highest point of the packing layer is within 5% of the set value of the height of a highest point of the packing layer. More preferably, the packing is conducted so that the difference is within 3% of the set value.

The E is 10% of a set value of the height of a highest point of the packing layer. The plate-type reactor used according to the invention is suitable for use in, for example, gas-phase catalytic reactions. As the packing material, use is usually made of a particulate catalyst or a mixture of a particulate catalyst and inert particles. In that application with that packing material, the difference between a set value of the height of a highest point of the packing layer and the found value of the height of the packing layer is preferably within 5% of the set value, more preferably within 3% thereof, from the standpoint of more evenly packing the packing material to thereby attain an even pressure during use and inhibit catalytic performance from decreasing. In case where the value of E exceeds 10%, there are cases where the catalyst layer in that application has an uneven pressure during the reaction and the reactant locally blows through the catalyst layer, resulting in a decrease in the conversion of the reactant and a decrease in the yield of a target product.

In case where the value of B is larger than E/tanθ, there is a possibility that the found value of the height of the packing layer is larger than the value of E. B may be any value not larger than E/tanθ. Although small values of B are preferred from the standpoint of improving the evenness of the packing material charged, there are cases where the number of charging sites increases and the operation of charging the packing material becomes troublesome. From the standpoint of the efficiency of the operation of charging the packing material, the value of B is preferably not smaller

than $0.5 \times E/\tan\theta$, more preferably not smaller than $0.75 \times E/\tan\theta$.

The charging of a packing material into the packing section can be conducted so as to obtain a packing layer having the set value. Such charging of a packing material into a packing section can be accomplished by determining that amount of the packing material which, when the packing material is charged into one packing section from the charging site, results in the set value of height when measured at the charging site and charging the packing material from the charging site in the charging amount determined. The amount of the packing material to be charged can be determined, for example, from the volume of the packing section, bulk density of the catalyst, angle of repose of the catalyst, and number of charging sites per packing section.

Alternatively, the charging of a packing material into the packing section can be accomplished by charging the packing material from the charging site until the actual height of a highest point of the packing layer reaches the set value of height. An end point of such charging of a packing material into a packing section can be decided by a method in which the set value of height is marked on a heat-transfer plate and the height of the packing layer being packed is visually watched from above the heat-transfer plate or monitored with a camera inserted into the space, or by a method in which use is made of a contact-type sensor which detects the position of the top of the packing layer ascending with the charging of the packing material, or by a method in which a rod is inserted from above and brought into contact with the top of the packing layer to determine the position of the top.

Whether the packing layer formed is satisfactory or not can be evaluated based on a comparison between the set value and the found value or a comparison of the packing heights of the packing material in each packing section. For example, the packing layer formed is satisfactory when the difference between the set value of the height of a highest point of the packing layer and the found value of a highest point thereof is 0-10% of the set value, and is more preferred when that difference is 0-5% of the set value.

Methods for charging the packing material into the packing section are not particularly limited. Examples of the methods include the charging of the packing material by a worker from charging sites and the charging of the packing material from charging sites with a device or apparatus for packing. There are cases where a device or apparatus for use in packing a packing material into the reaction tubes of a multitubular reactor can be used, as it is or after having been improved, as the device or apparatus for packing.

Such methods for charging the packing material into the packing section preferably are methods in which the rate of supplying the packing material to the packing section is controllable, and preferably are methods in which the amount of the packing material to be supplied to the packing section is controllable. From these standpoints, it is preferred to charge the packing material into the packing section with a packing apparatus which has a vessel such as a hopper and a conveying device the speed of which can be freely regulated, such as a belt conveyor.

In the case where the packing material is packed into the one packing section from a plurality of charging sites, the packing material is charged into the space so that the following expression (2) is satisfied besides expression (1). In the following expression (2), symbol A is the distance (m) between adjacent two of the charging sites and represents the distance between the adjacent side edges of respective adjacent flows of the packing material.

$$A \leq 2 \times E/\tan\theta \qquad (2)$$

In case where the value of A is larger than $2 \times E/\tan\theta$, there is a possibility that the found value of the height of the packing layer is larger than the value of E. The distance A may be any value not larger than $2 \times E/\tan\theta$. Although small values of A are preferred, as in the case of B, from the standpoint of improving the evenness of the packing material charged, there are cases where the number of charging sites increases and the operation of charging the packing material becomes troublesome. From the standpoint of the efficiency of the operation of charging the packing material, the value of A is preferably not smaller than $1 \times E/\tan\theta$, more preferably not smaller than $1.5 \times E/\tan\theta$.

In the case where a packing material is packed into one packing section from a plurality of charging sites, the packing material is simultaneously charged into the one packing section. The term "simultaneously charged" means that the packing material begins to be simultaneously charged from the charging sites in the same amount at the same rate. The amount of the packing material to be charged from each charging site and the rate of charging the packing material therefrom are not limited so long as the amount and rate are substantially the same. For example, the charging amount may be any value within $\pm 10\%$ based on the average of the charging amounts of the packing material charged from the respective charging sites. The charging rate may be any value so long as the charging period at the charging site where the charging has ended latest is within +20% based on the charging period at the charging site where the charging has ended earliest. Preferably, the former charging period is within +10% based on the latter.

The packing section is a chamber which is located in the space between the heat-transfer plates and is capable of holding the packing material therein. In the case where no partition for vertically separating the space has been disposed in the space, the packing section is the space. In the case where such partitions have been disposed in the space, the packing section is one of the sections formed by the partitions. The length of the packing section, in terms of length in the axial direction for the heat-transfer tubes, can be regulated by disposing such partitions in the space. When the length of each packing section, in terms of length in the axial direction for the heat-transfer tubes, is short, it is easy to reduce the number of packing-material charging sites and a packing material is apt to be evenly packed with ease. Consequently, from the standpoint of the efficiency of operation for evenly packing a packing material, the length of each packing section, in terms of length in the axial direction for the heat-transfer tubes, is preferably from 0.05 m to 2 m, more preferably 0.1-1 m, even more preferably 0.2-0.5 m.

The volume of each packing section surrounding by the heat-transfer plates and partitions is preferably 1-100 L, more preferably 1.5-30 L, especially preferably 2-15 L, from the standpoint that the packing of a packing material into the space is conducted independently with respect to the individual sections to enable the packing material to be precisely and easily packed.

As the packing material, a particulate packing material is used. One packing material or two or more packing materials may be used. When two or more packing materials are used, they may be packed as a mixture or in a stacked state. Packing materials are selected according to applications of the plate-type reactor. Examples of the packing material include particulate catalysts and inert particles which are in general use in gas-phase catalytic reactions. Examples of the catalysts include Mo-Bi composite oxide catalysts such as those described in JP-A-2005-336142 and Mo-V composite oxide catalysts. Examples of the inert particles include particles made of substances which are inert under use conditions for the plate-type reactor, such as mullite, alumina, silicon carbide, silica, zirconia, and titanium oxide.

The shape and size of the packing material are suitably selected from such a range that the packing material can be packed into the space between the heat-transfer plates. Examples of the shape of the packing material include spherical, solid-cylinder, hollow-cylinder, star, ring, small-piece, net, and indefinite shapes. In the case where the packing material is, for example, a catalyst or inert particles, the size of the packing material is preferably 1-20 mm, more preferably 3-10 mm, even more preferably 4-8 mm, in terms of maximum particle diameter.

The angle of repose is an index to the flowability of a packing material, and is expressed by the angle between the surface of the packing material which has flowed or is just before flowing and a horizontal plane. The smaller the angle of repose, the more the packing material is flowable and preferred from the standpoint of evenly packing the packing material into a packing section. From such standpoints, the angle of repose of the packing material is preferably 60 degrees or smaller, more preferably 55 degrees or smaller.

The angle of repose of a packing material can be regulated by mixing particulate materials differing in flowability. For example, in the case where the packing material is a particulate catalyst or inert particles, the flowability of this packing material can be heightened by mixing therewith inert particles having excellent flowability, such as mullite balls.

The angle of repose of a packing material can be measured, for exmple, by the so-called slope method, in which a cylindrical vessel containing a sufficient amount of the packing material is rolled at an appropriate speed and then stopped and the angle between the surface of the mass of the packing-material particles in the cylinder and a horizontal plane is determined as the angle of repose.

The plate-type reactor into which a packing material has been packed by the packing method can be used in a catalytic reaction in which a raw material having flowability and passing through the packing layer containing a particulate catalyst is reacted in the presence of the catalyst. Examples of the raw material include liquids and gases. Such catalytic reactions can be conducted based on known techniques relating to the catalyst, raw material, and reaction conditions to be used.

The plate-type reactor used according to the invention is suitable especially for the case where the raw material is a gas, from which heat removal is more difficult than liquids. Furthermore, the invention can be applied to a method for yielding a product through an exothermic gas-phase catalytic reaction or endothermic gas-phase catalytic reaction which is conducted in the presence of a particulate catalyst and is controllable with a heat medium flowing through heat-transfer tubes.

Examples of the exothermic gas-phase catalytic reaction include a gas-phase catalytic oxidation reaction in which either an unsaturated hydrocarbon or an unsaturated aldehyde corresponding thereto or both of these are oxidized to yield either the unsaturated aldehyde or the corresponding unsaturated carboxylic acid or both of these. More specific examples thereof include: a gas-phase catalytic oxidation reaction in which either acrolein and acrylic acid or methacrolein and methacrylic acid are yielded from propylene or isobutylene; and a gas-phase catalytic oxidation reaction in which acrylic acid or methacrylic acid is yielded from acrolein or methacrolein.

Other examples of the gas-phase catalytic reaction among exothermic catalytic reactions include: a reaction in which at least either one or more unsaturated aliphatic aldehydes having 3 to 4 carbon atoms or one or more unsaturated fatty acids having 3 to 4 carbon atoms are yielded from at least one member selected from the group consisting of hydrocarbons having 3 to 4 carbon atoms and tertiary butanol or at least one member selected from the group consisting of unsaturated aliphatic aldehydes having 3 to 4 carbon atoms and from oxygen; a reaction in which maleic acid is yielded from an aliphatic hydrocarbon having 4 or more carbon atoms and from oxygen; a reaction in which phthalic acid is yielded from o-xylene and oxygen; a reaction in which a paraffin is yielded by the hydrogenation of an olefin; a reaction in which butadiene is yielded by the oxidation/dehydrogenation of butene; a reaction in which ethylene oxide is yielded from ethylene and oxygen; and a reaction in which an alcohol is yielded by the hydrogenation of a carbonyl compound. Examples of liquid-phase catalytic reactions include a reaction in which acetone and phenol are yielded by decomposing cumene hydroperoxide with an acid.

Examples of the endothermic gas-phase catalytic reaction include a reaction in which styrene is yielded by the dehydrogenation of ethylbenzene.

In the process for producing a reaction product according to the invention, the plate-type reactor used according to the invention is used in the gas-phase catalytic reaction described above to produce methacrolein, acrolein, methacrylic acid, acrylic acid, maleic acid, phthalic acid, styrene, n-butene, isobutene, n-butane, isobutane, butadiene, or ethylene oxide by a gas-phase catalytic reaction in the presence of a particulate catalyst.

It is especially preferred that the plate-type reactor used according to the invention should be used in a gas-phase catalytic oxidation reaction in which either acrolein and acrylic acid or methacrolein and methacrylic acid are yielded from propylene or isobutylene and in a gas-phase catalytic oxidation reaction in which acrylic acid or methacrylic acid is yielded from acrolein or methacrolein. These reactions are highly exothermic gas-phase catalytic reactions in which cooling is important and the yield of the reaction product(s) can be considerably reduced by an increase in differential pressure caused by the breakage, powdering, etc. of the catalyst.

As the plate-type reactor, use can be made of a plate-type reactor which includes a reaction vessel for reacting one or more gaseous raw materials therein and a plurality of heat-transfer plates disposed side by side in the reaction vessel and in which the heat-transfer plates each include a plurality of heat-transfer tubes connected to each other at the periphery or edges of the sectional shape and have been disposed in the reaction vessel so that the heat-transfer tubes are connected to each other in the vertical direction. In this reactor, charging a particulate packing material into the space between adjacent heat-transfer plates gives a packing layer in the space.

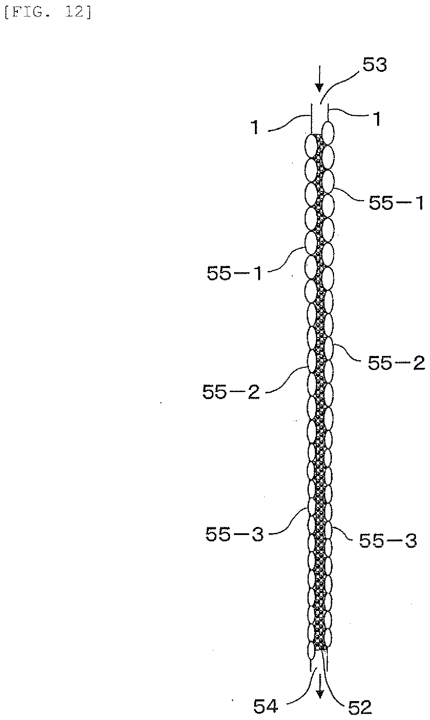

The plate-type reactor, for example, has the structure shown in Fig. 12. Namely, the plate-type reactor has heat-transfer plates 1 arranged face to face. The heat-transfer plates 1 have been disposed so that the axes of the heat-transfer plates 1 are vertically oriented and that the surface ridges of one of the heat-transfer plates 1 face the surface grooves of the other heat-transfer plate 1. By packing the space between the heat-transfer, plates 1 with a catalyst or a mixture of a catalyst and inert particles, a catalyst layer 52 is formed. A raw-material gas is fed to the catalyst layer 52 downward from above. Consequently, the upper end of the space between the opposed heat-transfer plates 1 is a gas inlet 53, and the lower end of the space is a gas outlet 54.

The heat-transfer plates 1 each have been configured so that the multiple heat-transfer tubes are connected to each other at the periphery of the sectional shape thereof. The heat-transfer tubes used include three kinds of heat-transfer tubes 55-1 to 55-3 differing in horizontal-direction radius. In the axial direction for the heat-transfer plate 1, the heat-transfer tubes 55-1, which are first in radius size, have been disposed in an upper part of the heat-transfer plate 1; the heat-transfer tubes 55-2, which are second in radius size, have been disposed in a middle part of the heat-transfer plate 1; and the heat-transfer tubes 55-3, which are last in radius size, have been disposed in a lower part of the heat-transfer plate 1.

In the catalyst layer 52 are formed: a first reaction zone, which is sandwiched between two sets of the heat-transfer tubes 55-1 and has a narrowest width and in which a raw-material gas fed comes first; a second reaction zone, which is sandwiched between two sets of the heat-transfer tubes 55-2 and has a second narrowest width and in which the gas that has passed through the first reaction zone comes; and a third reaction zone, which is sandwiched between two sets of the heat-transfer tubes 55-3 and has a largest width and in which the gas that has passed through the second reaction zone comes.

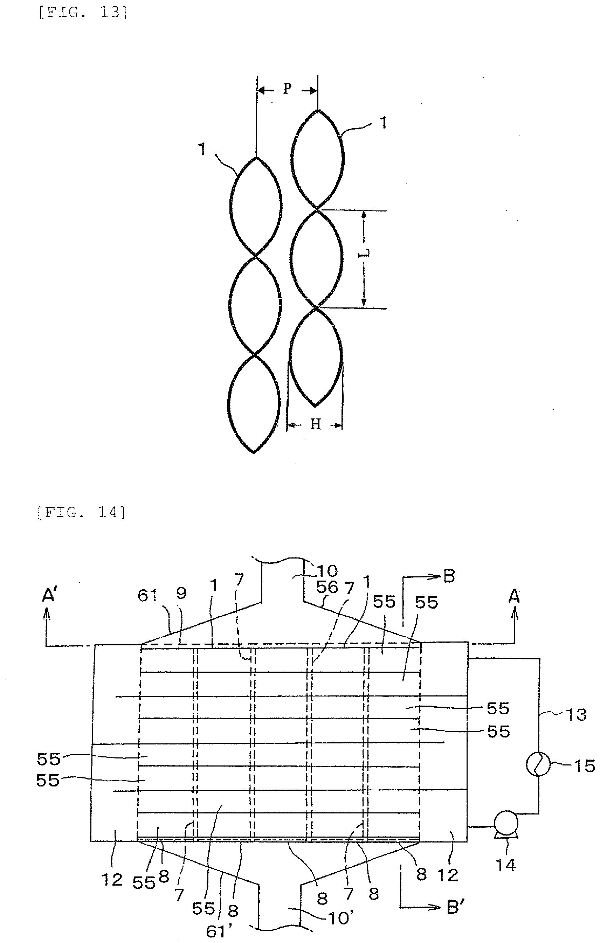

In Fig. 12, the opposed heat-transfer plates 1 have the same structure. With respect to the heat-transfer plates 1, as shown in Fig. 13, P denotes the distance between the axes of the respective heat-transfer plates, and H denotes the horizontal-direction diameter of the heat-transfer tubes (in heat-transfer tubes formed by bonding sheets which have been corrugated, H denotes the height of the corrugations). Furthermore, L denotes the vertical-direction diameter of the heat-transfer tubes (in heat-transfer tubes formed by bonding sheets which have been corrugated, L denotes the length of period of the corrugations).

As the reaction vessel, use can be made of a vessel which is suitable for the feeding of a gaseous raw material (raw-material gas) thereto and for the discharge of a product gas therefrom and in which a plurality of heat-transfer plates can be held side by side. Plate-type reactors are generally used for reactions conducted in high-pressure atmospheres. Because of this, the reaction vessel preferably is a pressure-resistant vessel capable of withstanding internal pressures of from ordinary pressure to 3 MPa (megapascals), preferably from ordinary pressure to 1,000 kPa (kilopascals), more preferably from ordinary pressure to 300 kPa. Examples of such a reaction vessel include: a shell constituted of a cylinder or a combination of parts of a cylinder; a shell the inside of which has been separated by one or more platy members so that a plurality of heat-transfer plates are held therein; and a vessel having a box-shaped internal cavity surrounded by members constituting flat inner surfaces so that a plurality of heat-transfer plates are held therein.

The number of heat-transfer plates can be determined based on the amount of the catalyst to be used for the reaction. From the standpoint of industrially producing a reaction product, the number thereof is preferably 10-300. The spacing between opposed heat-transfer plates can be determined based on the size of the packing material and the intended use of the plate-type reactor. For example, in the case where the plate-type reactor is for use in a gas-phase catalytic reaction, the spacing between the heat-transfer plates is preferably 10-50 mm, more preferably 20-35 mm, in terms of distance between the heat-transfer plates when the general size of particulate catalysts for use in gas-phase catalytic reactions is taken into account. The term "axis of a heat-transfer plate" has the following meanings. When all the heat-transfer tubes in the heat-transfer plate have been connected on one vertical line, that term means this vertical line. When not all of the connected parts of the heat-transfer tubes are on one vertical line, that term means a vertical line which passes through the horizontal-direction midpoint of these.

The heat-transfer plates each include a plurality of heat-transfer tubes connected to each other at the periphery or edges of the sectional shape. Namely, the heat-transfer plates are platy objects each including a plurality of heat-transfer tubes arranged in a row. In each heat-transfer plate, the heat-transfer tubes may have been directly connected to each other, or may have been indirectly connected to each other through an appropriate member such, e.g., a plate or hinges. From the standpoint of obtaining heat-transfer plates with high precision at low cost, it is preferred that each heat-transfer plate should be formed by: forming two steel sheets by press forming or roll forming into a shape which includes shapes corresponding to halves of the sectional shape of a heat-transfer tube and connected to each other directly or indirectly; and bonding the two formed steel sheets to each other. The heat-transfer plates may include only one kind of heat-transfer tubes or may include two or more kinds of heat-transfer tubes differing in sectional shape.

The heat-transfer plates are disposed in the reaction vessel so that the heat-transfer tubes are connected to each other in the vertical direction. In each heat-transfer plate, the angle between the axis of the heat-transfer tubes and the vertical direction is preferably 120-60 degrees, more preferably 100-80 degrees, even more preferably 90 degrees, from the standpoint that the state of reaction in the space between the heat-transfer plates is made even when the reactor is used in a gas-phase catalytic reaction.

Heat transfer between the gas flowing through the space between heat-transfer plates and the heat-transfer tubes is accelerated by the disturbance of gas flow caused by the corrugated surfaces of the heat-transfer plates due to the heat-transfer tubes. That angle is most preferably around 90 degrees from the standpoint of heat transfer. However, the pressure loss for the flowing gas is highest when that angle is 90 degrees. When the pressure loss for the gas is desired to be reduced, it is preferred to regulate that angle to a value other than 90 degrees. In this case, it is preferred to regulate the adjacent heat-transfer plates so as to have reversed angles, from the standpoint of homogenizing the flow of the gas and the catalyst being packed.

The vertical-direction (the direction in which a raw material passes) length of the heat-transfer plates disposed in the reaction vessel can be determined according to applications of the plate-type reactor. For example, when the reactor is for use in a gas-phase catalytic reaction, the vertical-direction length thereof is generally preferably 0.5-10 m, more preferably 0.5-5 m, even more preferably 0.5-3 m, from the standpoint of ensuring a length sufficient for yielding a reaction product. In view of the size of generally available steel sheets, two plates may be bonded to each other or used in combination when a vertical-direction'length of 1.5 m or larger is to be obtained.

The width of the heat-transfer plates (i.e., the length of the heat-transfer tubes) can be determined based on reaction conditions to be used in a gas-phase catalytic reaction. For example, the width of the heat-transfer plates is preferably 0.5-20 m, more preferably 3-15 m, even more preferably 6-10 m, from the standpoint of controlling reaction temperature with a heat medium flowing through the heat-transfer tubes.

The heat-transfer plates are constituted of a material having thermal conductivity. Examples of such a material include stainless steels, carbon steel, Hastelloy, titanium, aluminum, engineering plastics, and copper. It is preferred to use a stainless steel. Preferred of the stainless steels are 304, 304L, 316, and 316L.

The thickness of the sheets constituting each heat-transfer plate is preferably 2 mm or smaller, more preferably 1 mm or smaller.

The heat-transfer tubes are tubes through which a heat medium can pass and which have thermal conductivity. The sectional shape of the heat-transfer tubes is not particularly limited. Examples of the sectional shape of the heat-transfer tubes include a circular shape, approximately circular shapes such as elliptic shapes and a Rugby ball shape, a leaf shape composed of arcs symmetrically connected to each other, and polygonal shapes such as, e.g., rectangles. The term "periphery of the sectional shape of a heat-transfer tube" means the periphery of a circle. The term "edges of the sectional shape of a heat-transfer tube" means the major-axis ends of an approximately circular shape or corner edges of a polygonal shape.

The diameter of the heat-transfer tubes can be determined based on contact between the heat-transfer tubes and a packing material. For example, when the general particle diameters of particulate catalysts for use in gas-phase reactions are taken into account, the diameter of the heat-transfer tubes is preferably 5-100 mm. The minor-axis length of the heat-transfer tubes is preferably 5-50 mm, more preferably 10-30 mm. The major-axis length of the heat-transfer tubes is preferably 10-100 mm, more preferably 20-50 mm.

Preferred examples of plate-type reactors employing such heat-transfer plates include a reactor having a plurality of heat-transfer plates each obtained by disposing, face to face, two corrugated sheets which have been shaped so as to include the shape of a circular arc, elliptic arc, or part of a rectangle or polygon and bonding the ridges of one of the two corrugated sheets to the ridges of the other to form a plurality of heat-medium passages, the heat-transfer plates having been arranged so that the corrugated-sheet ridges of one of adjacent heat-transfer plates face the corrugated-sheet grooves of the other to form a catalyst layer having a given spacing.

The plate-type reactor used according to the invention may further have constituent elements other than the constituent elements described above. Examples of such other constituent elements include a heat-medium feeder for supplying a heat medium to the heat-transfer tubes, partitions vertically disposed in the space to separate the space in the vertical direction, and gas-permeable plugs which have gas permeability and freely removably close end parts of the sections.

The heat-medium feeder is a device for supplying a heat medium having a desired temperature to the heat-transfer tubes of the heat-transfer plates disposed in the reaction vessel. Use of a heat-medium feeder is preferred from the standpoint of precisely regulating a gas-phase catalytic reaction. Examples of the heat-medium feeder include a device having a circulation channel through which a heat medium is circulated between the heat-transfer tubes and the outside thereof and further having a temperature regulator which regulates the temperature of the heat medium flowing through the circulation channel. Examples of the temperature regulator include a heat exchanger and a heat-medium mixer for mixing the heat medium flowing through the circulation channel with a heat medium having a different temperature.

The partitions are members which are vertically disposed in the space between heat-transfer plates and are intended to prevent a packing material from leaking from the sections formed by the partitions. Use of partitions is preferred from the standpoint of evenly packing a packing material separately into the sections and thereby evenly and easily packing the space with the packing material. When the partitions further have such a degree of rigidity that the partitions retain their shape after a packing material has been packed into the sections, these partitions can be used as a spacer for maintaining the distance between the heat-transfer plates. Examples of the partitions include plates or sheets, rectangular rods, round rods, and nets made of stainless steels, carbon steel, Hastelloy, titanium, aluminum, engineering plastics, and copper; glass-wool; and ceramic plates.

The gas-permeable plugs are members for freely and independently opening/closing the vertical-direction end parts of the sections. Use of such gas-permeable plugs is preferred from the standpoint of easily discharging the packing material independently from the sections. Examples of the gas-permeable plugs include a member which has: a gas-permeable plate with which the end part of each section is covered; and a locking member which has been disposed on the gas-permeable plate and is capable of freely removably locking the gas-permeable plate to a heat-transfer plate or partition and of releasing the gas-permeable plate from the locked state when operated from above or below the section.

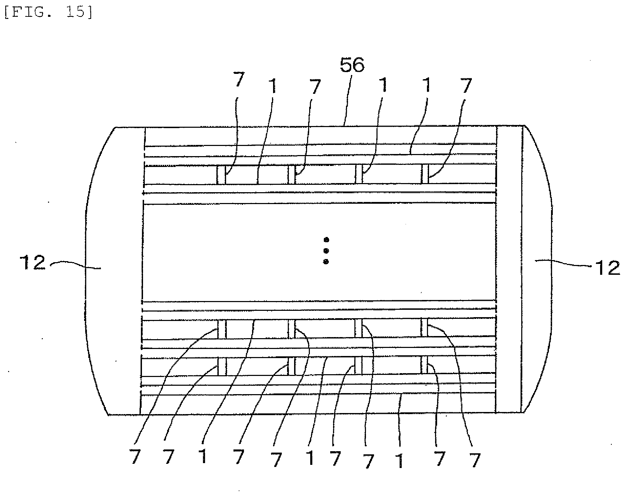

Examples of the plate-type reactor include a plate-type reactor which has, as shown in Fig. 14 to Fig. 16, a rectangular casing 56, a plurality of heat-transfer plates 1 having heat-transfer tubes 55 and disposed face to face in the casing 56, a heat-medium feeder for supplying a heat medium to the heat-transfer tubes 55, a plurality of partitions 7 which separate the space between adjacent heat-transfer plates 1 in the direction of gas passing in the casing 56 into a plurality of sections in which a packing material is to be packed and held, a plurality of gas-permeable plugs 8 which have gas permeability and with which the lower end part of each section is closed, and a perforated plate 9 disposed over the heat-transfer plates 1.

The casing 56 constitutes a gas passageway having a rectangular sectional shape, and corresponds to the reaction vessel. The casing 56 has a pair of opposed gas passage openings 10 and 10' respectively on the upper end and lower end of the casing 56. The casing 56 is constituted of a casing end part 61 having the gas passage opening 10, a casing end part 61' having the gas passage opening 10', and a casing main body for holding the heat-transfer plates 1 therein. The casing end parts 61 and 61' have been freely removably connected to the casing main body.

The heat-transfer plates 1 are the heat-transfer plates described above. The heat-transfer tubes 55 also are the heat-transfer tubes described above which are tubes having a leaf-shape section composed of two arcs symmetrically connected to each other at both ends and having thermal conductivity. As the heat-transfer tubes 55, three-kinds of heat-transfer tubes 55-1 to 55-3 are used as described above.

The heat-medium feeder has been disposed on a pair of opposed walls of the casing 56. These walls each have a feed opening for supplying a heat medium to the heat-transfer tubes 55. As shown in Fig. 14, the heat-medium feeder is constituted, for example, of a pair of jackets 12, a circulation channel 13 for circulating a heat medium inside and outside one of the jackets 12, a pump 14 disposed in the circulation channel 13, a heat exchanger 15 for regulating the temperature of the heat medium flowing through the circulation channel 13, and a heat-medium mixer for mixing a heat medium with the heat medium present in the jacket 12. The jackets 12 have been separated into sections at a given height so that the heat medium flows through the heat-transfer tubes 55 in zigzag directions between the jackets 12, for example, throughout the whole reaction vessel.

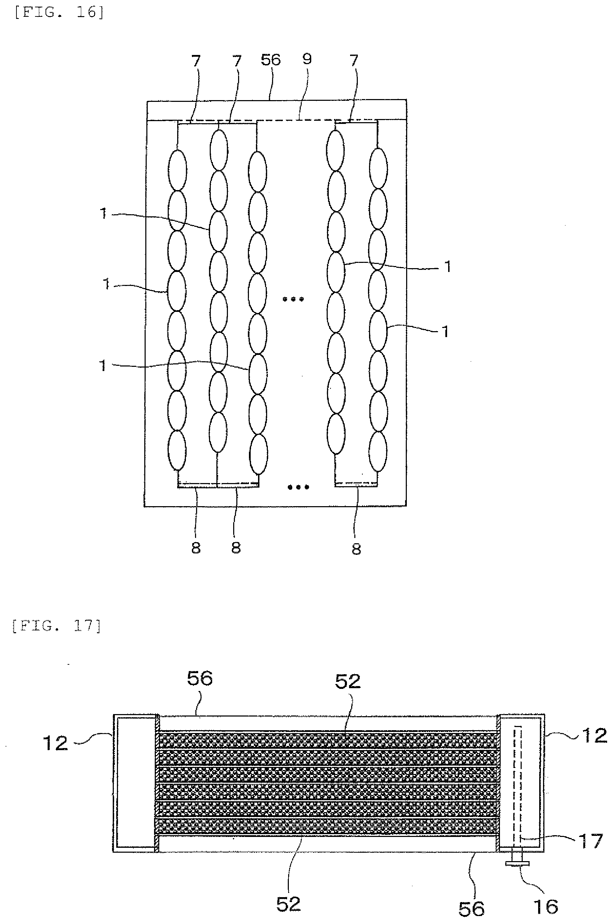

The heat-medium mixer has, as shown in, e.g., Fig. 17, a nozzle 16 which causes the inside and outside of the jacket 12 to communicate with each other and a distributing pipe 17 which has been connected to the nozzle 16 in the jacket 12 and extends in a direction perpendicular to the flow direction for the heat medium within the jacket 12. The distributing pipe 17 has a closed end and has a plurality of holes formed therein throughout the whole longitudinal direction for the distributing pipe.

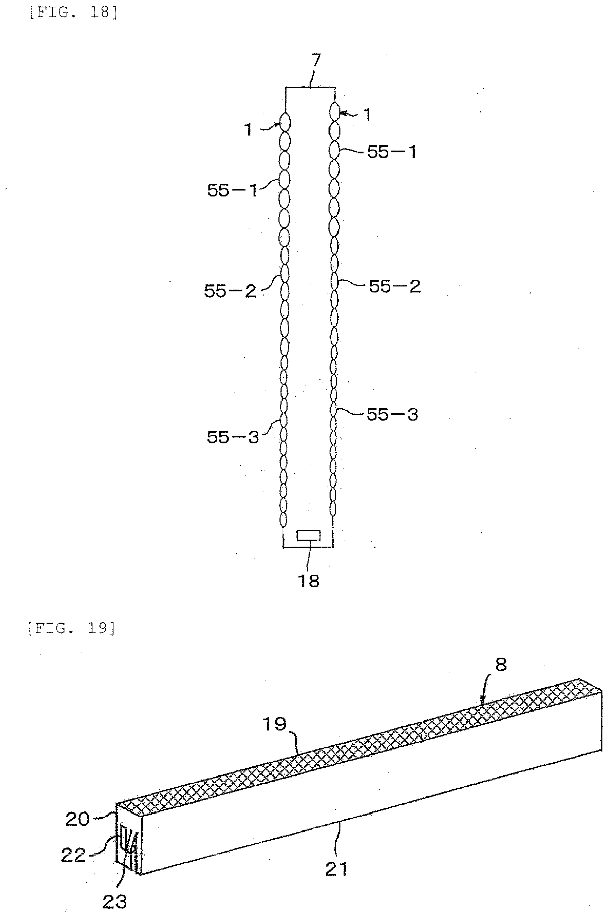

As shown in Fig. 18, the partitions 7 each are a stainless-steel sheet having side edges in close contact with the surface corrugations of the heat-transfer plates 1, and have an oblong rectangular window 18 in a lower end part thereof. The partitions 7 have been vertically disposed in the space between the opposed heat-transfer plates 1. The partitions 7 have been disposed at equal intervals so that sections of a given volume are formed in the space.

As shown in Fig. 19, each gas-permeable plug 8 has: a gas-permeable plate 19 having the same rectangular shape as the sectional shape of each section; first skirt parts 20 vertically extending downward from the short sides of the gas-permeable plate 19; and second skirt parts 21 vertically extending downward from the long sides of the gas-permeable plate 19. Each first skirt part 20 has a rectangular lock window 22 formed therein and a locking nib 23 disposed adjacently thereto.

The gas-permeable plate 19, for example, is a plate having 2-mm circular holes formed therein at a rate of openings of 30%. The lock window 22 has such a size that the width and height thereof are sufficient for housing the locking nib 23 therein. The locking nib 23 has been formed by making two parallel incisions from the lower edge of the first skirt part 20 and bending the incised area so as to form an outward projection. In the pair of opposed first skirt parts 20, the lock window 22 on one side faces the locking nib 23 on the other side, and the locking nib 23 on one side faces the lock window 22 on the other side. The window 18 of each partition 7 has such a size that the width and height thereof are sufficient for including both the lock window 22 and the locking nib 23 therein.

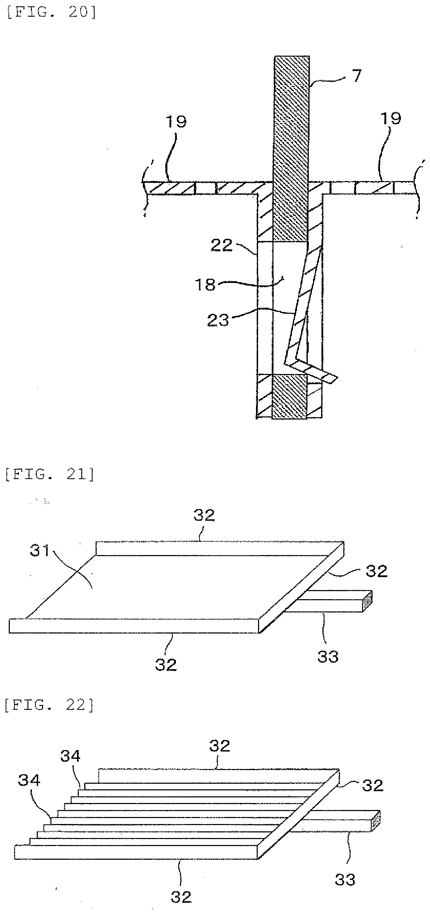

The gas-permeable plug 8 has been inserted into each section from the lower end of the section, with the gas-permeable plate 19 facing upward. When the gas-permeable plug 8 is inserted, the partition 7 presses the locking nib 23 while resisting the outward force thereof. However, when the locking nib 23 reaches the window 18, the nib 23 is released from the pressing with the partition 7 and protrudes into the window 18. The nib 23 thus locks into the window 18 as shown in Fig. 20.

The perforated plate 9, for example, is a plate having holes with a diameter which is 0.3-0.8 times the largest value of the major-axis length of the packing material to be packed, the holes having been formed at a rate of openings of 20-40%. The perforated plate 9 has been configured so that the space between the side edge of each of the outermost heat-transfer plates 1 and the wall of the casing 56 is closed, as shown in Fig. 14, in order to prevent a gas from passing through the space between the end of the outermost heat-transfer plate 1 and the wall of the casing 56.

In this plate-type reactor, the packing of a packing material into the space between heat-transfer plates 1 is accomplished by packing the packing material into each section from above the heat-transfer plates 1. The packing material is packed from charging sites which are located in desired positions according to distances from the end of each section in the vertical direction. In this packing of a packing material, the packing material is charged into the sections in an amount suitable for the volume of each section and the number of charging sites for charging the packing material into the section.

As the packing material, use may be made, for example, of an Mo-Bi composite oxide catalyst or Mo-V composite oxide catalyst for producing acrolein or acrylic acid through the gas-phase catalytic reaction of hydrocarbons such as propane and propylene and inert particles such as mullite, alumina, silicon carbide, silica, zirconia, or titanium oxide. When a catalyst and inert particles are used, the catalyst and the inert particles are used as a mixture thereof or in a stacked state. The content of the inert particles in the mixture or stack is, for example, 1-400 parts by mass per 100 parts by mass of the catalyst. The shape of the catalyst is, for example, a spherical, cylindrical, ring, or star shape or the like, and the shape of the inert particles is, for example, a spherical, cylindrical, ring, or star shape or the like.

When the distance from a charging site for a packing material to an end of a section in the axial direction for the heat-transfer tubes 55 (horizontal direction) is expressed by B (m), the angle of repose of the packing material is expressed by $\theta$ degree, and 10% of a reference value of the height of the packing layer to be formed in the section (set value of the height of a highest point of the catalyst layer) is expressed by E (m) and when the width of the section is 2B or smaller, i.e., not larger than $2 \times \tan\theta/E$, then the charging site can be in any desired position which is horizontally apart from either of the ends of the section at a distance of $\tan\theta/E$ or shorter.

When there is one charging site per packing section as described above, a packing material can be packed into each packing section in an amount suitable for the volume of the packing section. For example, such packing of a packing material can be accomplished by packing the packing material from a container, e.g., a beaker, at a suitable rate of 50-5,000 mL/min.

Alternatively, such packing of a packing material can be conducted with a packing device which, for example, has a rectangular plate 31, frames 32 rising respectively from three sides of the plate 31, and a handle 33 disposed on the frame 32 which is located at the central side of the three sides, as shown in Fig. 21. Examples of the material of this packing device include stainless steel.

The packing device may further have guide plates 34 for guiding a packing material from the central side to the opposite frame-free side, as shown in Fig. 22, the guide plates 34 having a height smaller than the maximum particle diameter of the packing material. The packing device may have only one guide plate 34, or may have two or more guide plates 34. The packing device having such guide plates 34 is preferred to the packing device shown in Fig. 21 from the standpoint of regulating the width of a flow of the packing material being charged into a packing section to the width of the open part (frame-free side) of the device.

When either of these packing devices is used for packing a packing material, the device can be used in the following manner. The packing material is placed on the plate 31, and the handle 33 is then held to shake the device from side to side and thereby level the packing material placed. The packing material is thus dispersed to such a degree that the packing-material particles are not stacked up vertically. Subsequently, the packing material is charged from the whole width of the open part into a packing section at a suitable rate of 50-5,000 mL/min while preventing the packing-material particles from being vertically stacked up.

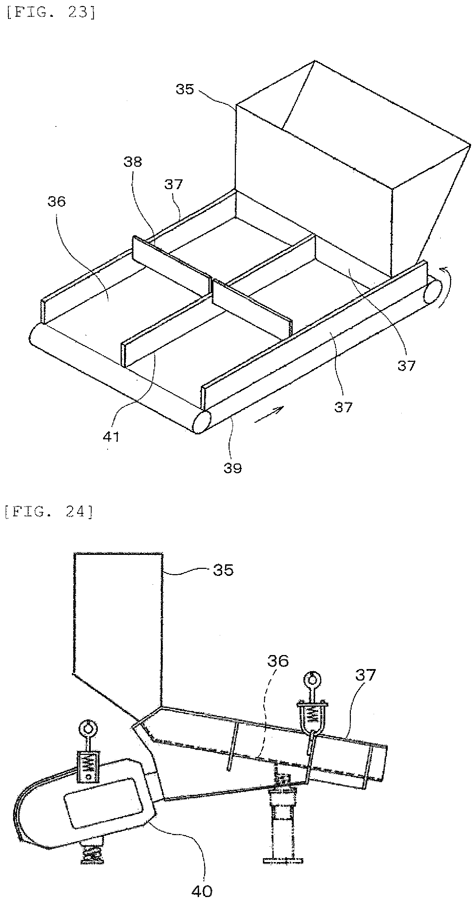

Alternatively, such packing of a packing material can be conducted with a packing device which, for example, has a hopper 35 for holding the packing material therein, a rectangular conveying surface 36 to which the packing material is supplied from the hopper 35, frames 37 disposed so as to rise respectively from three sides of the conveying surface 36, a control member 38 serving to regulate the height of the packing material being conveyed on the conveying surface 36 to a desired height, and a conveyor which conveys the packing material supplied onto the conveying surface 36 to the open part of the conveying surface 36 (the frame-free side of the conveying surface 36), as shown in Fig. 23.

Examples of the conveyor include a belt conveyor 39 which constitutes the conveying surface 36 and runs from the central side of the three sides, on which the hopper 35 has been disposed, toward the open part, as shown in Fig. 23. Examples thereof further include a vibrator 40 which, as shown in Fig. 24, vibrates a plate constituting a smooth conveying surface 36 which inclines from a hopper 35 toward an open part.

The packing device may further have a partition 41 which separates the conveying surface 36 in the conveying direction for the packing material, as shown in Fig. 23. Such a partition 41 is preferred from the standpoint of leveling the packing material on the conveying surface 36 in the width direction for the open part.

In the packing device, when the particles of the packing material being conveyed on the conveying surface 36 toward the open part are stacked up, the particles are regulates by the control member 38 and leveled in the width direction for the open part. The packing material is then charged from the open part into a packing section while flowing over the same width as the open part. The packing device shown in Fig. 23 is preferred from the standpoint of preventing the packing material from being broken, powdered, or otherwise damaged by conveyance, because the packing material is conveyed with a belt conveyor. Furthermore, the packing device shown in Fig. 24 has a simpler configuration because the packing material is conveyed with a vibrator. This device is hence preferred from the standpoint of the efficiency of the operation of packing a packing material into a plate-type reactor.

With respect to the open part in each of Fig.21 to Fig. 24, the width of the flow of the packing material being charged from the open part is usually equal to the width of the open part. As shown above, the width of the flow of a packing material can be changed by selecting a packing method or regulating the width of the open part. In the case where the width of the flow of a packing material is 0.05 m or smaller, the center of the width of the flow of the packing material, in terms of center in the axial direction for the heat-transfer tubes, is the charging site. When the width of the flow of a packing material exceeds 0.05 m, then the position of that one of the side edges of the flow of the packing material, in terms of side edges in the axial direction for the heat-transfer tubes, which is located on the side nearer to an end of the packing section being packed or which is located on the side nearer to an adjacent flow of the packing material is the charging site.

When the section has a width which is 2B or larger, i.e., $2\times\tan\theta/E$ or larger, then the charging sites can be in any desired two or more positions which are horizontally apart from the corresponding ends of the section at a distance of $\tan\theta/E$ or shorter and which satisfy that A is $2\times E/\tan\theta$ or shorter provided that A is the distance (m) between the adjacent charging sites.

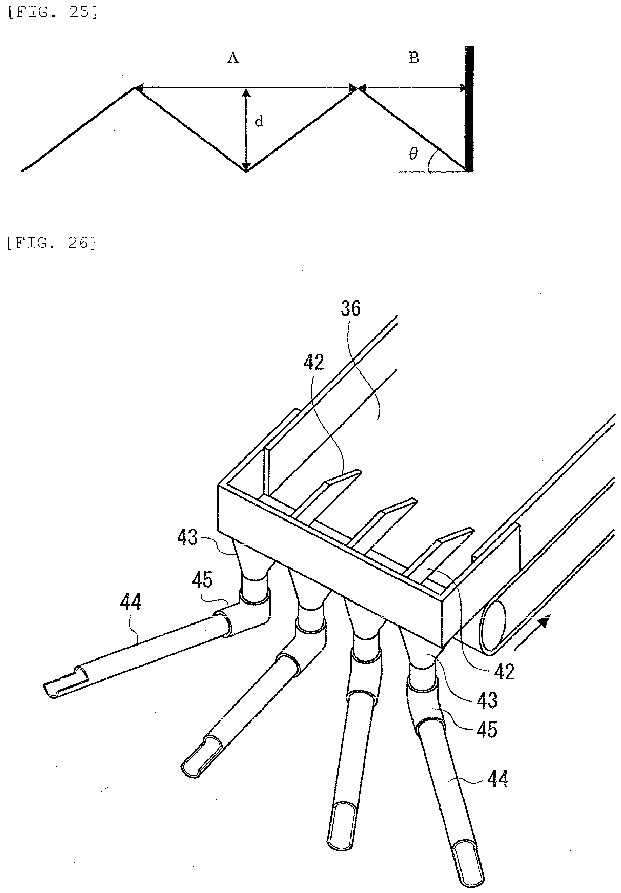

For example, when the distance between charging sites is expressed by A, the angle of repose of a packing material is expressed by $\theta$, and the difference in height in the top of the packing layer to be formed is expressed by d as shown in Fig. 25, then the value of d is represented by $A/2\times\tan\theta$. When a set value of the height of a highest point of the packing layer is expressed by L and height differences corresponding to up to 10% of the height of the highest point of the packing layer are allowable, then the value d is $0.1\times L$ or smaller. Consequently, the value of A is $0.1\times L\times2/\tan\theta$ or smaller. In the case where the set value of the height of a highest point of the packing layer is, for example, 1.7 m, then the value of A is 0.41 m or smaller when $\theta$ is 40 degrees, and the value of A is 0.29 m or smaller when $\theta$ is 50 degrees. The distance B between an end of the section and the charging site located nearest to the end of the section is regulated to A/2 or shorter.

In the case where there are two or more charging sites per packing section, a packing material is packed from the charging sites simultaneously. In such packing, when the volume of the packing section is constant, it is possible to charge the packing material simultaneously from the charging sites each in an amount obtained by dividing the volume of the packing section by the number of the charging sites. Alternatively, it is possible to charge the packing material simultaneously from the charging sites at a suitable desired rate of 50-5,000 mL/min.

Such packing of a packing material can be conducted using the packing device shown in Fig. 23 or Fig. 24 in combination with a distributor such as that shown in Fig. 26. This distributor has: a plurality of dividing plates 42 which equally divide the open part of a conveying surface 36 in the width direction; a plurality of funnels 43 to which a packing material divided into portions by the dividing plates 42 is supplied; and a plurality of feed pipes 44 for supplying the packing material divided into portions from the respective funnels 43 to desired charging sites for a desired packing section. Each feed pipe 44 is freely swingable in the horizontal direction due to the use of an elbow 45, and can be designed to have any desired length based on the use of a stretchable pipe or connection of a pipe having an appropriate length with a joint.

According to such packing of a packing material, an even packing layer can be easily formed in which the heights of the top of the packing layer are wholly included in the range of 90-100% of a found value of the height of a highest point of the packing layer.

Incidentally, as a set value for a packing layer, a set value of the height of any desired position other than the highest point of the packing layer may be used, such as the lowest point of the packing layer or the midpoint between the lowest point and the highest point. Even when a packing material is packed based on expressions obtained by converting the expression (1) and expression (2) based on that different set value, it is possible to easily form an even packing layer in which the heights of the top of the packing layer are wholly included in the range of 90-100% of a found value of the height of a highest point of the packing layer.

Furthermore, according to the packing of a packing material described above, when there are two or more charging sites per packing section, a packing material can be evenly packed so that a packing layer in which the positions of the top of the packing material are within an allowable range is formed in each section. Incidentally, the distributor can be used also in the charging of a packing material into a plurality of packing sections for which there is one charging site per packing section.

Moreover, since the plate-type reactor has partitions 7, the volume of each of the sections formed by the partitions 7 is far smaller than the space and, hence, a packing material can be easily packed evenly. As a result, this configuration is more effective from the standpoint of easily and evenly packing a packing material into all spaces.

Although the plate-type reactor shown in figures has partitions 7, a plate-type reactor having no partitions 7 can be packed in the following manner. Each space between heat-transfer plates 1 is taken as one packing section, and a packing material is charged into the packing section in the same manner as in the packing of a packing material into each section of the plate-type reactor described above.

The packing material can be discharged independently from the sections by removing each gas-permeable plug 8 from the space. Each gas-permeable plug 8 is removed by pushing each locking nib 23 from the lower end of the adjoining section to release the locking nib 23 from the state of being locked to the window 18. In this manner, the packing of a packing material can be easily redone with respect to each section. This method of packing is hence more effective from the standpoint of evenly and easily packing a packing material.

In the plate-type reactor packed with a packing material, a raw-material gas is fed to the space, for example, downward from above, and a heat medium is supplied to the heat-transfer tubes 55, whereby a reaction product is produced. For example, a raw-material gas composed of propylene, air, and water vapor is fed through the gas passage opening 10, and a heat medium having a temperature of 300-350°C is supplied from the heat-medium feeder to the heat-transfer tubes 55, for example, upward from below, whereby acrolein and acrylic acid are yielded. The acrolein and acrylic acid yielded are discharged through the gas passage opening 10'. The internal pressure of the plate-type reactor during the reaction is, for example, 150-200 kPa (kilopascals). The heat generated by the oxidation is absorbed by the heat medium flowing through the heat-transfer tubes 55.

In the production of acrolein and acrylic acid, the temperature of the heat medium is regulated by succeedingly passing the heat medium through the heat-transfer tubes 55, or with the heat exchanger 15, or by mixing a heat medium having a given temperature with the heat medium present in the jackets 12 by means of the heat-medium mixer. In the case where the raw reactant material is propylene, the temperature of the heat medium to be supplied to the multiple reaction zones is preferably 250-400°C, more preferably 320-400°C. On the other hand, in the case where the raw reactant material is acrolein, the temperature of the heat medium to be supplied to the multiple reaction zones is preferably 200-350°C, more preferably 250-320°C.

In the plate-type reactor, a packing layer constituted of an evenly packed packing material is formed in the space. Consequently, the reactor is inhibited from undergoing a phenomenon attributable to the packed state, i.e., packing density, of the packing material constituting the catalyst layer 52, such as the occurrence of a pressure loss in the packing layer and partial unevenness of the amount of a gas passing through the packing layer. The production of acrolein and acrylic acid can hence be stably conducted over long while maintaining an initial high efficiency.

EXAMPLES

<First Aspect of the Invention>

Examples are given below. However, the invention should not be construed as being limited by the following Examples unless the invention departs from the spirit thereof.

<Catalyst>

The catalyst used in the Examples was produced by the method disclosed in, e.g., JP-A-63-54942, JP-B-6-13096, and JP-B-6-38918. A metal oxide powder having the composition $Mo(12)Bi(5)Co(3)Ni(2)Fe(0.4)Na(0.4)B(0.2)K(0.08)Si(24)O(x)$ (the oxygen proportion x is a number determined according to the oxidized state of each metal so as to render the compound electrically neutral as a whole) was prepared as a catalyst powder. This catalyst powder was molded to produce a pellet-form catalyst having an outer diameter of 4 mm and a height of 3 mm.

<Reactor>

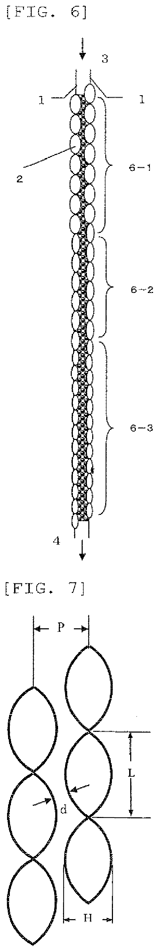

A reactor having the structure described in Figs. 6 and 7 was used in the Examples. Heat-transfer plates for the reactor each were obtained by bonding two corrugated stainless-steel sheets (sheet thickness, 1 mm) to each other to form heat-medium passages for reaction temperature regulation. Two of the bonded heat-transfer plates, which had heat-transfer tubes, were used as a pair to from a fixed catalyst bed.
In the reactor, the fixed catalyst bed has been divided into a reaction zone 6-1, a reaction zone 6-2, and a reaction zone 6-3 from the upstream side in the reactant-gas flow direction, according to the specifications of the corrugated sheet. The pair of bonded corrugated plates were located in parallel. The distance between the adjacent heat-transfer plates was 26 mm; the plate width was 114 mm; and the plate had a height of 1,810 mm. The period of the corrugations (L in Fig. 7), the height of the period of corrugations (H in Fig. 7), and the number of the corrugations are shown in Table 1. The minimum spacing d between the heat-transfer plates was 8 mm.

[Table 1]

Table 1

|  | Period (L) (mm) | Height (H) (mm) | Number of corrugations |
| --- | --- | --- | --- |
| First reaction zone | 40 | 20 | 15 |
| Second reaction zone | 40 | 16 | 11 |
| Third reaction zone | 30 | 10 | 23 |

<EXAMPLE 1>

Air having ordinary temperature was passed through the reactor packed with no catalyst at a space velocity, in terms of normal-state velocity per unit catalyst layer volume, of 7,200 (l/hr) while regulating the reactor outlet pressure to ordinary pressure and without conducting heating with a heat medium. Namely, air having ordinary temperature was passed through the reactor at a rate of 21,600 NL (indicating the number of liters of air in the normal state (0°C, 1 atm); the same applies hereinafter) per hour. During this passing, the difference in pressure between the raw-material gas inlet and outlet of the reactor was 30 Pa. This value of differential pressure was determined by placing water in a tube connecting the reactor inlet to the reactor outlet and measuring the difference in height between the resultant water columns.
On the other hand, the reactor was packed with the pellet-form catalyst (diameter, 4 mm ; height 3 mm; catalyst particle diameter D = 5 mm; bulk-state porosity, 44%). Air having ordinary temperature was passed through the reactor packed with the catalyst at a space velocity, in terms of normal-state velocity per unit catalyst layer volume, of 1,800 (l/hr) while regulating the reactor outlet pressure to ordinary pressure and without conducting heating with a heat medium. Namely, air having ordinary temperature was passed through the catalyst-packed reactor at a rate of 5,400 NL/hr. During this passing, the difference in pressure between the raw-material gas inlet and outlet of the reactor was 7kPa.

Each layer in the reactor was packed with the catalyst. A raw-material gas having the composition shown below was introduced into the reactor at a flow rate of 6,655 (NL/hr). During this operation, the raw-material inlet pressure in the reactor was 0.09 MPa-G (G indicates gauge pressure; the same applies hereinafter), and the outlet pressure was 0.047 MPa-G.

| | |
|---|---|
| PP (propylene) | 8.7% |
| Water | 7.0% |
| Oxygen | 14.0% |
| Nitrogen | 70.3% |

The gas resulting from the reaction was sampled and analyzed by gas chromatography.
The differential pressure during the reaction was 43 kPa. The conversion of the PP was 96.0%, and the yield of acrolein and acrylic acid was 94.5%. The results thereof are shown in Table 2.

<EXAMPLE 2>

The same catalyst as in Example 1 was packed into the same reactor as in Example 1. A raw-material gas having the same composition as in Example 1 was introduced into the reactor at a flow rate of 5,700 (NL/hr). During this operation, the raw-material inlet pressure in the reactor was 0.09 MPa-G, and the outlet pressure was 0.056 MPa-G.
The gas resulting from the reaction was sampled and analyzed by gas chromatography.
The differential pressure during the reaction was 34 kPa. The conversion of the PP was 96.9%, and the yield of acrolein and acrylic acid was 94.5%. The results thereof are shown in Table 2.

<EXAMPLE 3>

Each layer in the same reactor as in Example 1 was packed with a pellet-form catalyst (diameter, 3 mm ; height, 3 mm; catalyst particle diameter D = 4.2 mm; bulk-state porosity, 40%). Air having ordinary temperature was passed through the reactor packed with the catalyst at a space velocity, in terms of normal-state velocity per unit catalyst volume, of 1,800 (l/hr) while regulating the reactor outlet pressure to ordinary pressure and without conducting heating with a heat medium. Namely, air having ordinary temperature was passed through the catalyst-packed reactor at a rate of 5,400 NL/hr. During this passing, the difference in pressure between the raw-material gas inlet and outlet of the reactor was 14kPa.
The reactor was packed with the catalyst. A raw-material gas having the same composition as in Example 1 was introduced into the reactor at a flow rate of 6,655 (NL/hr). During this operation, the raw-material inlet pressure in the reactor was 0.13 MPa-G, and the outlet pressure was 0.067 MPa-G.
The gas resulting from the reaction was sampled and analyzed by gas chromatography.
The differential pressure during the reaction was 65 kPa. The conversion of the PP was 97.7%, and the yield of acrolein and acrylic acid was 92.3%. The results thereof are shown in Table 2.

<COMPARATIVE EXAMPLE 1>

A reactor which was a single-tube reactor of the type including a single tube packed with a catalyst and immersed in a heat medium was used in the Comparative Example. The reaction tube employed in the reactor was a cylindrical reaction tube having an inner diameter of 25 mm and a length of 3,200 mm. The reaction tube had a catalyst layer length of 2,800 mm. Air having ordinary temperature was passed through the reactor packed with no catalyst at a space velocity, in terms of normal-state velocity per unit catalyst layer volume, of 7,200 (l/hr) while regulating the reactor outlet pressure at ordinary pressure and without conducting heating with a heat medium. Namely, air having ordinary temperature was passed through the reactor at a rate of 9,896 NL/hr. During this passing, the difference in pressure between the raw-material gas inlet and outlet of the reactor was 80 Pa.
On the other hand, the reactor was packed with the same catalyst as in Example 1. Air having ordinary temperature was passed through the reactor packed with the catalyst at a space velocity, in terms of normal-state velocity per unit catalyst layer volume, of 1,800 (l/hr) while regulating the reactor outlet pressure to ordinary pressure and without conducting heating with a heat medium. Namely, air having ordinary temperature was passed through the catalyst-packed reactor at a rate of 2,474 NL/hr. During this passing, the difference in pressure between the raw-material gas inlet and outlet of the reactor was 15kPa.
The reactor was packed with the catalyst. A raw-material gas having the composition shown below was introduced into the reactor at a flow rate of 3,132 (NL/hr). During this operation, the raw-material inlet pressure in the reactor was 0.12 MPa-G, and the outlet pressure was 0.062 MPa-G.

| PP (propylene) | 8.7% |
| Water | 7.0% |
| Oxygen | 14.0% |
| Nitrogen | 70.3% |

The gas resulting from the reaction was sampled and analyzed by gas chromatography.

The differential pressure during the reaction was 58 kPa. The conversion of the PP was 91.7%, and the yield of acrolein and acrylic acid was 86.5%. The results thereof are shown in Table 2.

Incidentally, the temperature was further elevated in an attempt to heighten the PP conversion. However, the temperature of the catalyst layer rose abruptly and, hence, the operation was stopped.

[Table 2]

Table 2

| | Superficial differential pressure (Pa) | Differential pressure in catalyst-packed state (kPa) | Space velocity of raw-material gas (l/hr) | D/d | Porosity of bulk-state catalyst (%) | Length of reactor (m) | | Porosity of catalyst layer (%) | Heat-medium supply temperature | Differential pressure during reaction (kPa) | Conversion of PP (%) | Yield (%) |
|---|---|---|---|---|---|---|---|---|---|---|---|---|
| Example 1 | 30 | 7 | 2218 | 0.625 | 44 | first reaction zone | 0.60 | 60 | 359 | 43 | 96.0 | 94.5 |
| | | | | | | second reaction zone | 0.44 | 49 | 358 | | | |
| | | | | | | third reaction zone | 0.69 | 49 | 350 | | | |
| Example 2 | 30 | 7 | 1900 | 0.625 | 44 | first reaction zone | 0.60 | 60 | 353 | 34 | 96.9 | 94.5 |
| | | | | | | second reaction zone | 0.44 | 49 | 350 | | | |
| | | | | | | third reaction zone | 0.69 | 49 | 350 | | | |
| Example 3 | 30 | 14 | 2218 | 0.53 | 40 | first reaction zone | 0.60 | 52 | 353 | 65 | 97.7 | 92.3 |
| | | | | | | second reaction zone | 0.44 | 42 | 350 | | | |
| | | | | | | third reaction zone | 0.69 | 42 | 350 | | | |

EP 2 363 203 B1

26

(continued)

| | Superficial differential pressure (Pa) | Differential pressure in catalyst-packed state (kPa) | Space velocity of raw-material gas (l/hr) | D/d | Porosity of bulk-state catalyst (%) | Length of reactor (m) | | Porosity of catalyst layer (%) | Heat-medium supply temperature | Differential pressure during reaction (kPa) | Conversion of PP (%) | Yield (%) |
|---|---|---|---|---|---|---|---|---|---|---|---|---|
| Comp. 1 Ex. | 80 | 15 | 2278 | 0.2 | 44 | first reaction zone | - | 49 | 365 | 58 | 91.7 | 86.5 |
| | | | | | | second reaction zone | - | | | | | |
| | | | | | | third reaction zone | - | | | | | |

<Second Aspect >

[Production of Plate-Type Reactor]

A plate-type reactor having the structure shown in Fig. 12 was used. Heat-transfer plates were produced in the following manner. Stainless-steel sheets having a thickness of 1 mm each were formed into a shape having three kinds of corrugations so as to give heat-transfer plates which, after disposition, formed a first, second, and third reaction zone arranged in this order from the upstream side in the raw-material gas flow direction. Two of the corrugated stainless-steel sheets obtained were bonded to each other to form a heat-transfer plate having a plurality of heat-transfer tubes connected to each other which served as heat-medium passages for reaction temperature regulation. The period length (L), height (H), and number of the corrugations, which are shown in Fig. 13, are shown in Table 3.

[Table 3]

Table 3

|  | Period length of corrugations L (mm) | Height of corrugations H (mm) | Number of corrugations (-) |
|---|---|---|---|
| First reaction zone (S1) | 40 | 20 | 15 |
| Second reaction zone (S2) | 40 | 16 | 11 |
| Third reaction zone (S3) | 30 | 10 | 23 |

As shown in Fig. 12 and Fig. 13, a pair of heat-transfer plates shown in Table 3 were disposed in parallel at a distance of 26 mm (P shown in Fig. 13) so that the ridges of one of the heat-transfer plates faced the grooves of the other heat-transfer plate. Thus, a reactor was produced. The width of the heat-transfer plates (length of the heat-transfer tubes) was 1,000 mm.

[Preparation of Catalyst]

A metal oxide powder having the composition $Mo_{12}Bi_5Co_3Ni_2Fe_{0.4}Na_{0.4}B_{0.2}K_{0.08}Si_{24}O_x$ was prepared as a catalyst for use in catalytically oxidizing gas-phase propylene with molecular oxygen to convert the propylene into acrolein and acrylic acid. In the formula, "x" in the $O_x$ is a number determined by the oxidized state of each metal oxide. The powder obtained was molded to obtain cylindrical molded objects having an outer diameter of 4 mm and a height of 3 mm. The molded objects obtained were calcined at 510°C for 4 hours in the presence of air to obtain composite oxide catalyst A. On the other hand, the powder was molded to obtain ring-shaped molded objects having an outer diameter of 6 mm, inner diameter of 2 mm, and height of 6 mm. The molded objects obtained were calcined in the same manner to obtain catalyst B. The angle of repose of each catalyst obtained was determined by the method shown below.

[Method of Measuring Angle of Repose]

As a measuring apparatus, use was made of tricyclic cylinder-rotation-type surface angle measuring apparatus GFL-68, manufactured by Tsutsui Scientific Instruments Co., Ltd. Into a 500-mL vessel was placed 250 g of the catalyst. This vessel was put on the pedestal, and the cylinder was rotated at 10 rpm for 60 seconds and then rotated at a reduced rotation speed of 2 rpm for 10' seconds. The apparatus was then switched off. The angle between the horizontal direction and the catalyst surface located in a section crossing the axial direction for the cylinder was measured as the angle of repose. The angle of repose of catalyst A was 45 degrees, while the angle of repose of catalyst B was 52 degrees.

<EXAMPLE 4 (reference)>

Catalyst A was packed into the space between the heat-transfer plates in the plate-type reactor. The positions apart from an end of the heat-transfer plates at distances of 0.17 m, 0.50 m, and 0.83 m, respectively, in the axial direction for the heat-transfer tubes were set as charging sites (namely, the positions of charging sites were determined so that the distance from each end of the heat-transfer plates to the nearest charging site was 0.17 m and the distance between the other adjacent charging sites was 0.33 m). The catalyst was packed simultaneously from the charging sites at a rate of 200 mL/min using beakers so as to give a catalyst layer in which the highest point had a height of 1.73 m. The catalyst was thus packed into the space in a total amount of 24.9 L. After the packing, the height of the catalyst layer was measured at each of the positions apart from an end of the heat-transfer plates at distances of 0 m, 0.17 m, 0.335 m, 0.50 m, 0.665 m, 0.83 m, and 1 m, respectively.

Of the measured catalyst-layer heights, the height of the catalyst layer as measured at the 0.5-m measuring point was 1.73 m, which was the highest. This catalyst-layer height was taken as a reference (highest point), and the difference between the catalyst-layer height at the 0.50-m measuring point and the catalyst-layer height as measured at each of the other measuring points was determined. Incidentally, the height of the catalyst layer was measured by bringing a stainless-steel rod from above into contact with the top of the packing layer to determine the position of the top. The results thereof are shown in Table 4. The difference between the catalyst-layer height at the 0.50-m measuring point and the catalyst-layer height as measured at each of the other measuring points was within the range up to 0.173 m, i.e., within 10% of the catalyst-layer height of 1.73 m as measured at the 0.50-m measuring point.

[Table 4]

Table 4

| Measuring point [m] | 0 | 0.170 | 0.335 | 0.500 | 0.665 | 0.830 | 1.000 |
|---|---|---|---|---|---|---|---|
| Packing height [m] | 1.61 | 1.70 | 1.58 | 1.73 | 1.63 | 1.72 | 1.58 |
| Difference in height [m] | 0.12 | 0.03 | 0.15 | 0 | 0.10 | 0.01 | 0.15 |

<COMPARATIVE EXAMPLE 2>

Catalyst A was packed into the reactor in the same manner as in Example 4, except that charging sites were set at the positions apart from one end of the heat-transfer plates at distances of 0.25 m and 0.75 m (namely, the positions of charging sites were determined so that the distance from each end of the heat-transfer plates to the nearest charging site was 0.25 m and the distance between the other adjacent charging sites was 0.50 m). Catalyst A was packed simultaneously from the charging sites at a rate of 200 mL/min using beakers. The catalyst was thus packed into the space in a total amount of 24.1 L. After the packing, the height of the catalyst layer was measured at each of the positions apart from an end of the heat-transfer plates at distances of 0 m, 0.25 m, 0.50 m, 0.75 m, and 1 m, respectively.

Of the measured catalyst-layer heights, the height of the catalyst layer as measured at the 0.25-m measuring point was 1.73 m, which was the highest. This catalyst-layer height was taken as a reference (highest point), and the difference between the catalyst-layer height at the 0.25-m measuring point and the catalyst-layer height as measured at each of the other measuring points was determined. The results thereof are shown in Table 5. The difference between the catalyst-layer height at the 0.25-m measuring point and the catalyst-layer height as measured at each of the 0-m, 0.50-m, and 1-m measuring points was not within the range up to 0.173 m, i.e., not within 10% of the catalyst-layer height of 1.73 m as measured at the 0.25-m measuring point.

[Table 5]

Table 5

| Measuring point [m] | 0 | 0.25 | 0.50 | 0.75 | 1 |
|---|---|---|---|---|---|
| Packing height [m] | 1.44 | 1.73 | 1.49 | 1.71 | 1.45 |
| Difference in height [m] | 0.29 | 0 | 0.24 | 0.02 | 0.28 |

<EXAMPLE 5 (reference)>

Partitions were disposed in the space between the heat-transfer plates of the plate-type reactor at intervals of 0.5 m, and catalyst A was packed into one of the resultant sections. In preparation for the packing, charging sites were set at the positions apart from an end of the section at distances of 0.17 m and 0.33 m, respectively, so that the charging sites were apart from the corresponding ends of the section at a distance of 0.17 m and the distance between the two charging sites was 0.16 m. The catalyst was packed simultaneously from the charging sites at a rate of 200 mL/min using beakers. The catalyst was thus packed into the section in a total amount of 12.4 L. The height of the catalyst layer was measured at each of the positions apart from an end of the section at distances of 0 m, 0.17 m, 0.25 m, 0.33 m, and 0.50 m, respectively.

Of the measured catalyst-layer heights, the height of the catalyst layer as measured at the 0.17-m measuring point was 1.73 m, which was the highest. This catalyst-layer height was taken as a reference (highest point), and the difference between the catalyst-layer height as measured at the 0.17-m measuring point and the catalyst-layer height as measured at each of the other measuring points was determined. The results thereof are shown in Table 6. The difference between the catalyst-layer height at the 0.17-m measuring point and the catalyst-layer height as measured at each of the other measuring points was within the range up to 0.173 m, i.e., within 10% of the catalyst-layer height of 1.73 m as measured at the 0.17-m measuring point.

[Table 6]

Table 6

| Measuring point [m] | 0 | 0.17 | 0.25 | 0.33 | 0.50 |
|---|---|---|---|---|---|
| Packing height [m] | 1.59 | 1.73 | 1.63 | 1.72 | 1.58 |
| Difference in height [m] | 0.14 | 0 | 0.10 | 0.01 | 0.15 |

COMPARATIVE EXAMPLE 3>

Partitions were disposed in the space between the heat-transfer plates of the plate-type reactor at intervals of 0.5 m, and catalyst A was packed into one of the resultant sections. In preparation for the packing, a charging site was set at the position apart from one end of the section at a distance of 0.25 m so that the charging site was apart from each end of the section at a distance of 0.25 m. The catalyst was packed from the charging site at a rate of 200 mL/min using a beaker. The catalyst was thus packed into the section in a total amount of 11.9 L. The height of the catalyst layer was measured at each of the positions apart from an end of the section at distances of 0 m, 0.25 m, and 0.50 m, respectively.

The height of the catalyst layer as measured at the measuring point was 1.73 m. This catalyst-layer height was taken as a reference (highest point), and the difference between the catalyst-layer height as measured at this measuring point and the catalyst-layer height as measured at each of the other measuring points was determined. The results thereof are shown in Table 7. The difference between the catalyst-layer height at the 0.25-m measuring point and the catalyst-layer height as measured at each of the 0-m and 0.50-m measuring points was not within the range up to 0.173 m, i.e., not within 10% of the catalyst-layer height of 1.73 m as measured at the 0.25-m measuring point.

[Table 7]

Table 7

| Measuring point [m] | 0 | 0.25 | 0.50 |
|---|---|---|---|
| Packing height [m] | 1.42 | 1.73 | 1.43 |
| Difference in height [m] | 0.31 | 0 | 0.30 |

<EXAMPLE 6 (reference)>

Catalyst A was packed into the space between the heat-transfer plates of the plate-type reactor. The packing device shown in Fig. 22 which had an open-part width of 0.10 m was used for the packing in place of beakers.

Charging sites were set at the positions apart from one end of the space at distances of 0.116-0.216 m, 0.450-0.550 m, and 0.784-0.884 m, respectively. The catalyst was packed simultaneously from the charging sites at a rate of 200 mL/min. The catalyst was thus packed into the space in a total amount of 25.1 L. The height of the catalyst layer was measured at each of the positions apart from an end of the space at distances of 0 m, 0.166 m, 0.333 m, 0.500 m, 0.666 m, 0.834 m, and 1 m, respectively.

Of the measured catalyst-layer heights, the height of the catalyst layer as measured at the 0.500-m measuring point was 1.73 m, which was the highest. This catalyst-layer height was taken as a reference (highest point), and the difference between the catalyst-layer height as measured at the 0.500-m measuring point and the catalyst-layer height as measured at each of the other measuring points was determined. The results thereof are shown in Table 8. The difference between the catalyst-layer height at the 0.500-m measuring point and the catalyst-layer height as measured at each of the other measuring points was within the range up to 0.173 m, i.e., within 10% of the catalyst-layer height of 1.73 m as measured at the 0.500-m measuring point.

[Table 8]

Table 8

| Measuring point [m] | 0 | 0.166 | 0.333 | 0.500 | 0.667 | 0.834 | 1 |
|---|---|---|---|---|---|---|---|
| Packing height [m] | 1.63 | 1.72 | 1.60 | 1.73 | 1.63 | 1.72 | 1.65 |
| Difference in height [m] | 0.10 | 0.01 | 0.15 | 0 | 0.10 | 0.01 | 0.08 |

<COMPARATIVE EXAMPLE 4>

Catalyst A was packed into the space between the heat-transfer plates of the plate-type reactor using the same packing device as in Example 6. Charging sites were set at the positions apart from an end of the space at distances of 0.2-0.3 m and 0.7-0.8 m, respectively. The catalyst was packed simultaneously from the charging sites at a rate of 200 mL/min. The catalyst was thus packed into the space in a total amount of 24.3 L. The height of the catalyst layer was measured at each of the positions apart from an end of the space at distances of 0 m, 0.25 m, 0.50 m, 0.75 m, and 1 m, respectively.

Of the measured catalyst-layer heights, the height of the catalyst layer as measured at the 0.25-m measuring point was 1.73 m, which was the highest. This catalyst-layer height (highest point) was taken as a reference, and the difference between the catalyst-layer height at the 0.25-m measuring point and the catalyst-layer height as measured at each of the other measuring points was determined. The results thereof are shown in Table 9. The difference between the catalyst-layer height at the 0.25-m measuring point and the catalyst-layer height as measured at each of the 0-m, 0.50-m, and 1-m measuring points was not within the range up to 0.173 m, i.e., not within 10% of the catalyst-layer height of 1.73 m as measured at the 0.25-m measuring point.

[Table 9]

Table 9

| Measuring point [m] | 0 | 0.25 | 0.50 | 0.75 | 1 |
|---|---|---|---|---|---|
| Packing height [m] | 1.50 | 1.73 | 1.52 | 1.72 | 1.48 |
| Difference in height - [m] | 0.22 | 0 | 0.20 | 0.01 | 0.24 |

<EXAMPLE 7 (reference)>

Catalyst B was packed into the space between the heat-transfer plates in the plate-type reactor. The positions apart from an end of the heat-transfer plates at distances of 0.125 m, 0.375 m, 0.625 m, and 0.875 m, respectively, in the axial direction for the heat-transfer tubes were :set as charging sites (namely, the positions of charging sites were determined so that the distance from each end of the heat-transfer plates to the nearest charging site was 0.125 m and the distance between the other adjacent charging sites was 0.25 m). The catalyst was packed simultaneously from the charging sites at a rate of 200 mL/min using beakers so as to give a catalyst layer in which the highest point had a height of 1.73 m. The catalyst was thus packed into the space in a total amount of 25.0 L. After the packing, the height of the catalyst layer was measured at each of the positions apart from an end of the heat-transfer plates at distances of 0 m, 0.125 m, 0.250 m, 0.375 m, 0.500 m, 0.625 m, 0.750 m, 0.875 m, and 1 m, respectively.

Of the measured catalyst-layer heights, the height of the catalyst layer as measured at the 0.875-m measuring point was 1.73 m, which was the highest. This catalyst-layer height was taken as a reference (highest point), and the difference between the catalyst-layer height at the 0.875-m measuring point and the catalyst-layer height as measured at each of the other measuring points was determined. The results thereof are shown in Table 10. The difference between the catalyst-layer height at the 0.875-m measuring point and the catalyst-layer height as measured at each of the other measuring points was within the range up to 0.173 m, i.e., within 10% of the catalyst-layer height of 1.73 m as measured at the 0.875-m measuring point.

[Table 10]

Table 10

| Measuring point [m] | 0 | 0.125 | 0.250 | 0.375 | 0.500 | 0.625 | 0.750 | 0.875 | 1 |
|---|---|---|---|---|---|---|---|---|---|
| Packing height [m] | 1.63 | 1.70 | 1. 60 | 1.71 | 1.63 | 1.70 | 1.65 | 1.73 | 1.62 |
| Difference in height [m] | 0.10 | 0.03 | 0.13 | 0.02 | 0.10 | 0.03 | 0.08 | 0 | 0.11 |

<COMPARATIVE EXAMPLE 5>

Catalyst B was packed into the space between the heat-transfer plates in the plate-type reactor. The positions apart from an end of the heat-transfer plates at distances of 0.17 m, 0.50 m, and 0.83 m, respectively, in the axial direction for the heat-transfer tubes were set as charging sites (namely, the positions of charging sites were determined so that the distance from each end of the heat-transfer plates to the nearest charging site was 0.17 m and the distance between the other adjacent charging sites was 0.33 m) . The catalyst was packed simultaneously from the charging sites at a rate of 200 mL/min using beakers so as to give a catalyst layer in which the highest point had a height of 1.73 m. The catalyst was thus packed into the space in a total amount of 24.2 L. After the packing, the height of the catalyst layer was measured at each of the positions apart from an end of the heat-transfer plates at distances of 0 m, 0.170 m, 0.335 m, 0.500 m, 0.665 m, 0.830 m, and 1 m, respectively.

Of the measured catalyst-layer heights, the height of the catalyst layer as measured at the 0.500-m measuring point was 1.73 m, which was the highest. This catalyst-layer height was taken as a reference (highest point), and the difference between the catalyst-layer height at the 0.500-m measuring point and the catalyst-layer height as measured at each of the other measuring points was determined. The results thereof are shown in Table 11. The difference between the catalyst-layer height at the 0.500-m measuring point and the catalyst-layer height as measured at each of the 0-m, 0.335-m, 0.665-m, and 1-m measuring points was not within the range up to 0.173 m, i.e., not within 10% of the catalyst-layer height of 1.73 m as measured at the 0.500-m measuring point.

[Table 11]

Table 11

| Measuring point [m] | 0 | 0.170 | 0.335 | 0.500 | 0.665 | 0.830 | 1 |
|---|---|---|---|---|---|---|---|
| Packing height [m] | 1.43 | 1.69 | 1.51 | 1.73 | 1.52 | 1.70 | 1.48 |
| Difference in height [m] | 0.30 | 0.04 | 0.22 | 0 | 0.21 | 0.03 | 0.25 |

The following was found from Examples 4 to 7 and Comparative Examples 2 to 5 given above. When a catalyst is packed into the space from a plurality of sites satisfying expression (1) and expression (2), differences in catalyst-layer height in the axial direction for the heat-transfer tubes can be reduced and an even catalyst layer can be formed. On the other hand, when a catalyst is packed into the space from sites satisfying neither expression (1) nor expression (2), the resultant catalyst layer has increased differences in height in the axial direction for the heat-transfer tubes and the catalyst is not evenly packed.

While the invention has been described in detail and with reference to specific embodiments thereof, it will be

**EP 2 363 203 B1**

apparent to one skilled in the art that various changes and modifications can be made therein without departing from the scope of the claims.

This application is based on a Japanese patent application filed on November 25, 2008 (Application No. 2008-299895) and a Japanese patent application filed on December 26, 2008 (Application No. 2008-334079), the contents thereof being herein incorporated by reference.

INDUSTRIAL APPLICABILITY

Shell-and-tube reactors (reference) and plate-type reactors (invention) are excellent from the standpoint of efficiently producing products in large quantities generally through gas-phase reactions. By the method of reaction according to the first aspect of the invention, products can be highly efficiently produced in high yield. The suitability of shell-and-tube reactors and plate-type reactors for general-purpose use is expected to be enhanced further.

The space between heat-transfer plates in plate-type reactors is generally small, and have a flat and complicated shape. Because of this, when a packing material is packed into the space between heat-transfer plates, it is generally difficult to ascertain or put right the state of the packing. However, according to the second aspect, a packing material can be properly packed into the space between heat-transfer plates, and such a properly packed state can be easily ascertained. An improvement in periodic operations in plate-type reactors, such as safety inspections, is therefore expected. In the production of products with plate-type reactors, a further improvement in long-term productivity including suitability for such periodic operations is expected. Consequently, the invention has a significant industrial value.

DESCRIPTION OF THE REFERENCE NUMERALS AND SIGNS

1 Heat-transfer plate
2 Heat-medium passage
3 Space
4 Reactant-gas inlet
5 Reactant-gas outlet
6-1 First reaction zone
6-2 Second reaction zone
6-3 Third reaction zone
11 Corrugated sheet
a Corrugated-sheet ridge
b Corrugated-sheet groove
P, P1, P2 Distance between plates (spacing between axes of pair of heat-transfer plates)
L Period of corrugations (period length of corrugations)
H Height of corrugations (Height of corrugations)
d, d1, d2, d3 Minimum spacing between two adjacent heat-transfer plates
52 Catalyst layer
53 Gas inlet
54 Gas outlet
55, 55-1, 55-2, 55-3 Heat-transfer tube
56 Casing
7 Partition
8 Gas-permeable plug
9 Perforated plate
10, 10' Gas passage opening
61, 61' Casing end part
12 Jacket
13 Circulation channel
14 Pump
15 Heat exchanger
16 Nozzle
17 Distributing pipe
18 Window
19 Gas-permeable plate
20 First skirt part

21 Second skirt part
22 Lock window
23 Locking nib
31 Plate
32, 37 Frame
33 Handle
34 Guide plate
35 Hopper
36 Conveying surface
38 Control member
39 Belt conveyor
40 Vibrator
41 Partition plate
42 Dividing plate
43 Funnel
44 Feed pipe
45 Elbow

**Claims**

1. A method of reaction which comprises using a reactor which is equipped with a catalyst layer for reacting a raw-material gas feed and is capable of cooling and heating through heat exchange with the catalyst layer, wherein the reactor, in the state of comprising no catalyst layer, has a differential pressure of 50 Pa or lower when air having 0 °C is introduced into the reactor at a space velocity, in terms of normal-state velocity per unit catalyst layer volume, of 7,200 1/hr while regulating the reactor outlet pressure to 1 atm and without conducting heating with a heat medium, the catalyst layer having been divided into a plurality of reaction zones arranged from a raw-material gas inlet to a raw-material gas outlet, the multiple reaction zones having been disposed so that the porosity thereof, which refers to the proportion of that part of the catalyst-packed space which is not occupied by the catalyst, as measured at the time of catalyst packing, decreases from the raw-material gas inlet toward the raw-material gas outlet,
   wherein the reactor is a plate-type reactor which is equipped with a catalyst layer formed between adjacent heat-transfer plates and in which a raw-material gas feed passes through the space between the adjacent heat-transfer plates and is then discharged, a catalyst layer being formed between the adjacent heat-transfer plates, and wherein the reactor is a plate-type reactor in which the catalyst layer formed between the adjacent heat-transfer plates has been divided into a plurality of reaction zones arranged from the raw-material gas inlet toward the raw-material gas outlet, and the multiple reaction zones increase in catalyst layer thickness, in terms of thickness in the direction of the length of the space between the adjacent heat-transfer plates, from the raw-material gas inlet toward the raw-material gas outlet, the ratio of the particle diameter (D) of the catalyst to be packed into the space between the adjacent heat-transfer plates to the minimum spacing (d) between the adjacent heat-transfer plates (D/d) satisfying 0.9>D/d>0.1,
   wherein the reactor is a reactor which, in the state of being equipped with a catalyst layer, has a differential pressure of 15 kPa or lower when air having 0 °C is introduced into the reactor at a space velocity, in terms of normal-state velocity per unit volume of the catalyst layer, of 1,800 1/hr while regulating the reactor outlet pressure to 1 atm and without conducting heating with a heat medium;
   wherein adjacent heat-transfer plates are disposed so as to be parallel with each other; and
   wherein "normal-state" refers to the conditions of 0°C and 1 atm,
   the term "thickness of the catalyst layer" refers to the average of catalyst-layer thicknesses as measured in the direction of the length of the space between heat-transfer plates in the reaction zone, and
   the term "particle diameter (D) of a catalyst" refers to the diameter when the catalyst has a spherical shape, refers to the major-axis length when the catalyst has a pellet shape, refers to the larger of the outer diameter of the circle and the height when the catalyst has a cylindrical or ring shape, refers to the outer diameter of the circle when the catalyst has a disk shape, refers to the dimension of the longest distance between two points on the periphery of a section formed by cutting the shape perpendicularly to the thickness direction when the catalyst has a platy shape, and refers to the axial-direction length when the catalyst has a rod shape.

2. The method of reaction as claimed in claim 1, wherein the catalyst to form the catalyst layer is a catalyst which, in a bulk state, has a porosity of 60% or lower.

3. The method of reaction as claimed in claim 1 or 2, wherein the raw-material gas is introduced into the reactor having a catalyst layer at a space velocity of from 1,100 1/hr to 4,200 1/hr, in terms of normal-state velocity per unit volume of the catalyst layer, to react the gas.

4. The method of reaction as claimed in any one of claims 1 to 3, wherein the raw-material gas is introduced into the reactor having a catalyst layer at a space velocity of from 1,800 l/hr to 4,200 l/hr, in terms of normal-state velocity per unit volume of the catalyst layer, to react the gas.

5. The method of reaction as claimed in any one of claims 1 to 4, which is used for:

producing (meth)acrolein and (meth)acrylic acid from propylene or isobutylene as a raw material; producing (meth)acrylic acid from (meth)acrolein as a raw material; producing ethylene oxide by oxidizing ethylene; producing either at least one unsaturated aliphatic aldehyde having 3 to 4 carbon atoms or at least one unsaturated fatty acid having 3 to 4 carbon atoms or both of these two kinds of compounds by oxidizing at least one member selected from the group consisting of hydrocarbons having 3 to 4 carbon atoms, tertiary butanol, and unsaturated aliphatic aldehydes having 3 to 4 carbon atoms;
producing maleic acid from an aliphatic hydrocarbon having 4 or more carbon atoms or an aromatic hydrocarbon as a raw material; producing phthalic acid by oxidizing o-xylene; or producing butadiene by oxidizing and dehydrogenating butene.

**Patentansprüche**

1. Ein Reaktionsverfahren, welches die Verwendung eines Reaktors umfasst, der mit einer Katalysatorschicht zum Umsetzen einer Rohmaterialgaszufuhr ausgestattet ist und durch Wärmeaustausch mit der Katalysatorschicht kühlen und erwärmen kann, wobei der Reaktor, in dem Zustand, in welchem er keine Katalysatorschicht umfasst, einen Differentialdruck von 50 Pa oder weniger aufweist, wenn Luft mit 0° C mit einer Raumgeschwindigkeit, bezogen auf die Geschwindigkeit im Normalzustand pro Einheit Katalysatorschichtvolumen, von 7200 l/h in den Reaktor eingeführt wird, unter Regelung des Reaktoraustrittsdrucks auf 1 atm und ohne Durchführen von Erwärmen mit einem Heizmedium, wobei die Katalysatorschicht in eine Mehrzahl von Reaktionszonen, angeordnet von einem Rohmaterialgas-Einlass zu einem Rohmaterialgas-Auslass, geteilt ist, wobei die mehreren Reaktionszonen derart angeordnet sind, dass deren Porosität, bezogen auf den Anteil des Teils des katalysatorgepackten Raums, der vom Katalysator nicht belegt ist, gemessen zum Zeitpunkt der Katalysatorpackung, von dem Rohmaterialgas-Einlass zum Rohmaterialgas-Auslass hin abnimmt,
wobei der Reaktor ein Reaktor vom Platten-Typ ist, welcher mit einer Katalysatorschicht ausgestattet ist, welche zwischen benachbarten Wärmeübertragungsplatten gebildet ist und in dem eine Rohmaterialgaszufuhr durch den Raum zwischen den benachbarten Wärmeübertragungsplatten hindurchströmt und dann abgegeben wird, wobei eine Katalysatorschicht zwischen den benachbarten Wärmeübertragungsplatten gebildet ist, und
wobei der Reaktor ein Reaktor vom Platten-Typ ist, in welchem die zwischen den benachbarten Wärmeübertragungsplatten gebildete Katalysatorschicht in eine Mehrzahl von Reaktionszonen unterteilt wurde, die vom Rohmaterialgas-Einlass zum Rohmaterialgas-Auslass hin angeordnet sind, und die Katalysatorschichtdicke in den mehreren Reaktionszonen, bezogen auf die Dicke in Richtung der Länge des Raumes zwischen den benachbarten Wärmeübertragungsplatten, vom Rohmaterialgas-Einlass zum Rohmaterialgas-Auslass hin zunimmt, wobei das Verhältnis des Teilchendurchmessers (D) des Katalysators, welcher in den Raum zwischen den benachbarten Wärmeübertragungsplatten gepackt werden soll, zum Mindestabstand (d) zwischen den benachbarten Wärmeübertragungsplatten (D/d), 0,9>D/d>0.1 erfüllt, wobei der Reaktor ein Reaktor ist, welcher, in dem Zustand, in welchem er mit einer Katalysatorschicht ausgestattet ist, einen Differentialdruck von 15 kPa oder weniger aufweist, wenn Luft mit 0° C mit einer Raumgeschwindigkeit, bezogen auf die Geschwindigkeit im Normalzustand pro Einheit Katalysatorschichtvolumen, von 1800 l/h in den Reaktor eingeführt wird, unter Regelung des Reaktoraustrittsdrucks auf 1 atm und ohne Durchführen von Erwärmen mit einem Heizmedium;
wobei benachbarte Wärmeübertragungsplatten derart angeordnet sind, dass sie parallel zueinander sind; und
wobei "Normalzustand" sich auf Bedingungen von 0° C und 1 atm bezieht,
der Begriff "Dicke der Katalysatorschicht" sich auf den Durchschnitt der Katalysatorschichtdicken, gemessen in Richtung der Länge des Raums zwischen den Wärmeübertragungsplatten in der Reaktionszone, bezieht und
der Begriff "Teilchendurchmesser (D) eines Katalysators" sich auf den Durchmesser bezieht, wenn der Katalysator eine Kugelform aufweist, auf die Hauptachsenlänge bezieht, wenn der Katalysator eine Pelletform aufweist, sich auf den größeren des äußeren Durchmesser des Kreises und die Höhe bezieht, wenn der Katalysator eine Zylinderform oder Ringform aufweist, sich auf den äußeren Durchmesser des Kreises bezieht, wenn der Katalysator eine Scheibenform aufweist, sich auf die Abmessung der längsten Distanz zwischen zwei Punkten auf der Peripherie eines Abschnitts, gebildet durch Schneiden der Form senkrecht zur Dickenrichtung bezieht, wenn der Katalysator eine plattenförmige Form aufweist und sich auf die Länge in axialer Richtung bezieht, wenn der Katalysator eine Stabform aufweist.

**2.** Das Reaktionsverfahren wie in Anspruch 1 beansprucht, wobei der Katalysator, welcher die Katalysatorschicht bildet, ein Katalysator ist, welcher, in einem Massezustand, eine Porosität von 60 % oder weniger aufweist.

**3.** Das Reaktionsverfahren wie in Anspruch 1 oder 2 beansprucht, wobei das Rohmaterialgas in den Reaktor, welcher eine Katalysatorschicht aufweist, mit einer Raumgeschwindigkeit von 1100 l/h bis 4200 l/h eingespeist wird, bezogen auf die Geschwindigkeit im Normalzustand pro Einheit Katalysatorschichtvolumen, um das Gas umzusetzen.

**4.** Das Reaktionsverfahren wie in einem der Ansprüche 1 bis 3 beansprucht, wobei das Rohmaterialgas in den Reaktor, welcher eine Katalysatorschicht aufweist, mit einer Raumgeschwindigkeit von 1800 l/h bis 4200 l/h eingespeist wird, bezogen auf die Geschwindigkeit im Normalzustand pro Einheit Katalysatorschichtvolumen, um das Gas umzusetzen.

**5.** Das Reaktionsverfahren wie in einem der Ansprüche 1 bis 4 beansprucht, welches verwendet wird zur: Herstellung von (Meth)acrolein und (Meth)acrylsäure aus Propylen oder Isobutylen als ein Rohmaterial; Herstellung von (Meth)acrylsäure aus (Meth)acrolein als ein Rohmaterial; Herstellung von Ethylenoxid durch Oxidieren von Ethylen; Herstellung von entweder mindestens einem ungesättigten aliphatischen Aldehyd mit 3 bis 4 Kohlenstoffatomen oder mindestens einer ungesättigten Fettsäure mit 3 bis 4 Kohlenstoffatomen oder beider dieser zwei Arten von Verbindungen durch Oxidieren mindestens eines Elements ausgewählt aus der Gruppe bestehend aus Kohlenwasserstoffen mit 3 bis 4 Kohlenstoffatomen, tertiärem Butanol und ungesättigten aliphatischen Aldehyden mit 3 bis 4 Kohlenstoffatomen; Herstellung von Maleinsäure aus einem aliphatischen Kohlenwasserstoff mit 4 oder mehr Kohlenstoffatomen oder einem aromatischen Kohlenwasserstoff als ein Rohmaterial; Herstellung von Phthalsäure durch Oxidieren von o-Xylen; oder Herstellung von Butadien durch Oxidieren und Dehydrieren von Buten.

## Revendications

**1.** Procédé de réaction qui comprend l'utilisation d'un réacteur qui est équipé d'une couche de catalyseur pour faire réagir une alimentation en gaz de matière première et est capable de refroidir et chauffer par échange de chaleur avec la couche de catalyseur, dans lequel le réacteur, dans l'état ne comprenant pas de couche de catalyseur, présente une pression différentielle de 50 Pa ou inférieure lorsque de l'air ayant 0°C est introduit dans le réacteur à une vitesse spatiale, en termes de vitesse à l'état normal par volume unitaire de couche de catalyseur, de 7 200 l/h tout en régulant la pression de sortie du réacteur à 1 atm et sans réaliser de chauffage avec un milieu de chauffage, la couche de catalyseur ayant été divisée en plusieurs zones de réaction disposées à partir d'une entrée de gaz de matière première vers une sortie de gaz de matière première, les multiples zones de réaction étant disposées de sorte que leur porosité, qui fait référence à la proportion de la partie de l'espace garni de catalyseur qui n'est pas occupée par le catalyseur, comme mesurée au moment du garnissage de catalyseur, diminue de l'entrée de gaz de matière première vers la sortie de gaz de matière première,
dans lequel le réacteur est un réacteur de type à plaque qui est équipé d'une couche de catalyseur formée entre des plaques de transfert de chaleur adjacentes et dans lequel une alimentation en gaz de matière première passe à travers l'espace entre les plaques de transfert de chaleur adjacentes et est ensuite déchargée, une couche de catalyseur étant formée entre les plaques de transfert de chaleur adjacentes, et dans lequel le réacteur est un réacteur de type à plaque dans lequel la couche de catalyseur formée entre les plaques de transfert de chaleur adjacentes a été divisée en plusieurs zones de réaction disposées à partir de l'entrée de gaz de matière première vers la sortie de gaz de matière première, et les multiples zones de réaction augmentent en épaisseur de couche de catalyseur, en termes d'épaisseur dans la direction de la longueur de l'espace entre les plaques de transfert de chaleur adjacentes, de l'entrée de gaz de matière première vers la sortie de gaz de matière première, le rapport du diamètre de particule (D) du catalyseur à garnir dans l'espace entre les plaques de transfert de chaleur adjacentes par rapport à l'espacement minimal (d) entre les plaques de transfert de chaleur adjacentes (D/d) satisfaisant 0,9>D/d>0,1,
dans lequel le réacteur est un réacteur qui, dans l'état où il est équipé d'une couche de catalyseur, présente une pression différentielle de 15 kPa ou inférieure lorsque de l'air ayant 0°C est introduit dans le réacteur à une vitesse spatiale, en termes de vitesse à l'état normal par volume unitaire de la couche de catalyseur, de 1 800 l/h tout en régulant la pression de sortie de réacteur à 1 atm et sans réaliser de chauffage avec un milieu de chauffage ;

dans lequel des plaques de transfert de chaleur adjacentes sont disposées afin d'être parallèles les unes aux autres ; et

dans lequel l'"état normal" fait référence aux conditions de 0°C et 1 atm, le terme "épaisseur de la couche de catalyseur" fait référence à la moyenne des épaisseurs de couche de catalyseur comme mesurées dans la direction de la longueur de l'espace entre les plaques de transfert de chaleur dans la zone de réaction, et

le terme "diamètre de particule (D) d'un catalyseur" fait référence au diamètre lorsque le catalyseur présente une forme sphérique, fait référence à la longueur d'axe principal lorsque le catalyseur présente une forme de boulette, fait référence à la valeur supérieure du diamètre externe du cercle et de la hauteur lorsque le catalyseur présente une forme cylindrique ou d'anneau, fait référence au diamètre externe du cercle lorsque le catalyseur présente une forme de disque, fait référence à la dimension de la distance la plus longue entre deux points sur la périphérie d'une section formée en découpant la forme perpendiculairement à la direction d'épaisseur lorsque le catalyseur présente une forme de plaque, et fait référence à la longueur de direction axiale lorsque le catalyseur présente une forme de barre.

2. Procédé de réaction selon la revendication 1, dans lequel le catalyseur pour former la couche de catalyseur est un catalyseur qui, dans un état de vrac, présente une porosité de 60 % ou inférieure.

3. Procédé de réaction selon la revendication 1 ou 2, dans lequel le gaz de matière première est introduit dans le réacteur présentant une couche de catalyseur à une vitesse spatiale de 1 100 l/h à 4 200 l/h, en termes de vitesse à l'état normal par volume unitaire de la couche de catalyseur, pour faire réagir le gaz.

4. Procédé de réaction selon l'une quelconque des revendications 1 à 3, dans lequel le gaz de matière première est introduit dans le réacteur ayant une couche de catalyseur à une vitesse spatiale de 1800 l/h à 4 200 l/h, en termes de vitesse à l'état normal par volume unitaire de la couche de catalyseur, pour faire réagir le gaz.

5. Procédé de réaction selon l'une quelconque des revendications 1 à 4, qui est utilisé pour : produire de la (méth)acroléine et de l'acide (méth)acrylique à partir de propylène ou d'isobutylène comme une matière première ; produire de l'acide (méth)acrylique à partir de (méth)acroléine comme une matière première ; produire de l'oxyde d'éthylène par oxydation d'éthylène ; produire soit au moins un aldéhyde aliphatique insaturé ayant de 3 à 4 atomes de carbone soit au moins un acide gras insaturé ayant de 3 à 4 atomes de carbone soit les deux de ces deux types de composés par oxydation d'au moins un élément choisi dans le groupe consistant en hydrocarbures ayant de 3 à 4 atomes de carbone, butanol tertiaire, et aldéhydes aliphatiques insaturés ayant de 3 à 4 atomes de carbone ; produire de l'acide maléique à partir d'un hydrocarbure aliphatique ayant 4 atomes de carbone ou plus ou d'un hydrocarbure aromatique comme une matière première ; produire de l'acide phtalique par oxydation d'o-xylène ; ou produire du butadiène par oxydation et déshydrogénation de butène.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

[FIG. 19]

[FIG. 20]

[FIG. 21]

[FIG. 22]

[FIG. 23]

[FIG. 24]

[FIG. 25]

[FIG. 26]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003267912 A
- JP 2004202430 A
- EP 1063222 A1
- EP 1526123 A1
- EP 1577001 A1
- JP 2005336142 A
- JP 63054942 A
- JP 6013096 B
- JP 6038918 B
- JP 2008299895 A
- JP 2008334079 A